# EUROPEAN PATENT APPLICATION

(11) **EP 2 636 738 A1**
(43) Date of publication of application: **11.09.2013**
(21) Application number: 11838039.3
(22) Date of filing: 01.11.2011
(51) Int. Cl.: C12N 15/09, C12N 1/19, C12P 19/14

(54) **TRANSFORMANT OF YEAST OF GENUS SCHIZOSACCHAROMYCES AND METHOD FOR PRODUCING SAME**

(30) Priority: 24.03.2011 JP 2011066540; 05.11.2010 JP 2010249092
(71) Applicant: Asahi Glass Company, Limited, Tokyo 100-8405 (JP)
(72) Inventor: TOHDA, Hideki, Tokyo 100-8405 (JP); OKADA, Katsunori, Tokyo 100-8405 (JP)
(74) Representative: Müller-Boré & Partner Patentanwälte
(86) International application number: PCT/JP2011/075220
(87) International publication number: WO 2012/060389

(57) **Abstract**

To provide a transformant of a yeast of the genus *Schizosaccharomyces* which can produce *β*-glucosidase, and a method for producing such a transformant.

A transformant of a yeast of the genus *Schizosaccharomyces* characterized by having a structural gene sequence encoding a *β*-glucosidase derived from a filamentous fungus, and a promoter sequence and a terminator sequence for expressing the structural gene in a chromosome, or alternatively by having the sequences as an extrachromosomal gene. Further, a transformation method for a yeast of the genus *Schizosaccharomyces,* characterized in that the yeast of the genus *Schizosaccharomyces* is transformed by using a vector having a structural gene sequence encoding a *β*-glucosidase derived from a filamentous fungus, and a promoter sequence and a terminator sequence for expressing the structural gene.

SEQUENCE LISTING
<110> ASAHIGLASS COMPANY, LIMITED
<120> Beta-glucosidase
<130> F20110368
<160> 4

<210> 1
<211> 841
<212> PRT
<213> Aspergillus aculeatus
<223> BGL1
<400> 1
Asp Glu Leu Ala Phe Ser Pro Pro Phe Tyr Pro Ser Pro Trp Ala Asn
                5                   10                  15
Gly Gln Gly Glu Trp Ala Glu Ala Tyr Gln Arg Ala Val Ala Ile Val
            20                  25                  30
Ser Gln Met Thr Leu Asp Glu Lys Val Asn Leu Thr Thr Gly Thr Gly
        35                  40                  45
Trp Glu Leu Glu Lys Cys Val Gly Gln Thr Gly Gly Val Pro Arg Leu
    50                  55                  60
Asn Ile Gly Gly Met Cys Leu Gln Asp Ser Pro Leu Gly Ile Arg Asp
65                  70                  75                  80
Ser Asp Tyr Asn Ser Ala Phe Pro Ala Gly Val Asn Val Ala Ala Thr
                85                  90                  95

Trp Asp Lys Asn Leu Ala Tyr Leu Arg Gly Gln Ala Met Gly Gln Glu
            100                 105                 110
Phe Ser Asp Lys Gly Ile Asp Val Gln Leu Gly Pro Ala Ala Gly Pro
        115                 120                 125
Leu Gly Arg Ser Pro Asp Gly Gly Arg Asn Trp Glu Gly Phe Ser Pro
    130                 135                 140
Asp Pro Ala Leu Thr Gly Val Leu Phe Ala Glu Thr Ile Lys Gly Ile
145                 150                 155                 160
Gln Asp Ala Gly Val Val Ala Thr Ala Lys His Tyr Ile Leu Asn Glu
                165                 170                 175
Gln Glu His Phe Arg Gln Val Ala Glu Ala Ala Gly Tyr Gly Phe Asn
            180                 185                 190
Ile Ser Asp Thr Ile Ser Ser Asn Val Asp Asp Lys Thr Ile His Glu
        195                 200                 205
Met Tyr Leu Trp Pro Phe Ala Asp Ala Val Arg Ala Gly Val Gly Ala
    210                 215                 220
Ile Met Cys Ser Tyr Asn Gln Ile Asn Asn Ser Tyr Gly Cys Gln Asn
225                 230                 235                 240
Ser Tyr Thr Leu Asn Lys Leu Leu Lys Ala Glu Leu Gly Phe Gln Gly
                245                 250                 255
Phe Val Met Ser Asp Trp Gly Ala His His Ser Gly Val Gly Ser Ala
            260                 265                 270
Leu Ala Gly Leu Asp Met Ser Met Pro Gly Asp Ile Thr Phe Asp Ser
        275                 280                 285
Ala Thr Ser Phe Trp Gly Thr Asn Leu Thr Ile Ala Val Leu Asn Gly
    290                 295                 300
Thr Val Pro Gln Trp Arg Val Asp Asp Met Ala Val Arg Ile Met Ala
305                 310                 315                 320
Ala Tyr Tyr Lys Val Gly Arg Asp Arg Leu Tyr Gln Pro Pro Asn Phe
                325                 330                 335
Ser Ser Trp Thr Arg Asp Glu Tyr Gly Phe Lys Tyr Phe Tyr Pro Gln
            340                 345                 350
Glu Gly Pro Tyr Glu Lys Val Asn His Phe Val Asn Val Gln Arg Asn
        355                 360                 365
His Ser Glu Val Ile Arg Lys Leu Gly Ala Asp Ser Thr Val Leu Leu
    370                 375                 380
Lys Asn Asn Asn Ala Leu Pro Leu Thr Gly Lys Glu Arg Lys Val Ala
385                 390                 395                 400

Ile Leu Gly Glu Asp Ala Gly Ser Asn Ser Tyr Gly Ala Asn Gly Cys
                405                 410                 415
Ser Asp Arg Gly Cys Asp Asn Gly Thr Leu Ala Met Ala Trp Gly Ser
            420                 425                 430
Gly Thr Ala Glu Phe Pro Tyr Leu Val Thr Pro Glu Gln Ala Ile Gln
        435                 440                 445
Ala Glu Val Leu Lys His Lys Gly Ser Val Tyr Ala Ile Thr Asp Asn
    450                 455                 460
Trp Ala Leu Ser Gln Val Glu Thr Leu Ala Lys Gln Ala Ser Val Ser
465                 470                 475                 480
Leu Val Phe Val Asn Ser Asp Ala Gly Glu Gly Tyr Ile Ser Val Asp
                485                 490                 495
Gly Asn Glu Gly Asp Arg Asn Asn Leu Thr Leu Trp Lys Asn Gly Asp
            500                 505                 510
Asn Leu Ile Lys Ala Ala Ala Asn Asn Cys Asn Asn Thr Ile Val Val
        515                 520                 525
Ile His Ser Val Gly Pro Val Leu Val Asp Glu Trp Tyr Asp His Pro
    530                 535                 540
Asn Val Thr Ala Ile Leu Trp Ala Gly Leu Pro Gly Gln Glu Ser Gly
545                 550                 555                 560
Asn Ser Leu Ala Asp Val Leu Tyr Gly Arg Val Asn Pro Gly Ala Lys
                565                 570                 575
Ser Pro Phe Thr Trp Gly Lys Thr Arg Glu Ala Tyr Gly Asp Tyr Leu
            580                 585                 590
Val Arg Glu Leu Asn Asn Gly Asn Gly Ala Pro Gln Asp Asp Phe Ser
        595                 600                 605
Glu Gly Val Phe Ile Asp Tyr Arg Gly Phe Asp Lys Arg Asn Glu Thr
    610                 615                 620
Pro Ile Tyr Glu Phe Gly His Gly Leu Ser Tyr Thr Thr Phe Asn Tyr
625                 630                 635                 640
Ser Gly Leu His Ile Gln Val Leu Asn Ala Ser Ser Asn Ala Gln Val
                645                 650                 655
Ala Thr Glu Thr Gly Ala Ala Pro Thr Phe Gly Gln Val Gly Asn Ala
            660                 665                 670
Ser Asp Tyr Val Tyr Pro Glu Gly Leu Thr Arg Ile Ser Lys Phe Ile
        675                 680                 685
Tyr Pro Trp Leu Asn Ser Thr Asp Leu Lys Ala Ser Ser Gly Asp Pro
    690                 695                 700
Tyr Tyr Gly Val Asp Thr Ala Glu His Val Pro Glu Gly Ala Thr Asp
705                 710                 715                 720
Gly Ser Pro Gln Pro Val Leu Pro Ala Gly Gly Gly Ser Gly Gly Asn
                725                 730                 735
Pro Arg Leu Tyr Asp Glu Leu Ile Arg Val Ser Val Thr Val Lys Asn
            740                 745                 750
Thr Gly Arg Val Ala Gly Asp Ala Val Pro Gln Leu Tyr Val Ser Leu
        755                 760                 765
Gly Gly Pro Asn Glu Pro Lys Val Val Leu Arg Lys Phe Asp Arg Leu
    770                 775                 780
Thr Leu Lys Pro Ser Glu Glu Thr Val Trp Thr Thr Thr Leu Thr Arg
785                 790                 795                 800
Arg Asp Leu Ser Asn Trp Asp Val Ala Ala Gln Asp Trp Val Ile Thr
                805                 810                 815
Ser Tyr Pro Lys Lys Val His Val Gly Ser Ser Ser Arg Gln Leu Pro
            820                 825                 830
Leu His Ala Ala Leu Pro Lys Val Gln
        835                 840

<210> 2
<211> 2664
<212> DNA
<213> Artificial Sequence
<220>
<223> Description of Artificial Sequence: modified AaBGL1 gene

<400> 2
cgaattggcg gaaggccgtc aaggccgcat ggtacctcat gaagttgtcc tggcttgagg 60
ccgctgccct taccgccgcc tccgtcgttt ccgccgacga gttggccttc tcccccccct 120
tctacccctc cccctgggcc aacggtcaag gtgagtgggc cgaggcttac caacgtgccg 180
tcgccattgt ctcccaaatg acccttgacg agaaggtcaa ccttaccacc ggtactggtt 240
gggagcttga gaagtgcgtc ggtcaaaccg gtggtgtccc ccgtcttaac atcggtggta 300
tgtgccttca agactccccc cttggtatcc gtgactccga ctacaactcc gccttccctg 360
ccggtgtcaa cgtcgccgcc acctgggaca agaaccttgc ctaccttcgt ggtcaagcta 420
tgggtcaaga gttctccgac aagggtatcg acgtccaact tggtcctgcc gccggtcccc 480
ttggtcgttc ccccgacggt ggtcgtaact gggagggttt ctcccccgac cccgccctta 540
ccggtgtcct tttcgccgag actatcaagg gtatccaaga cgctggtgtc gtcgccaccg 600
ccaagcacta catccttaac gagcaagagc acttccgtca agtcgccgag gccgctggtt 660
acggtttcaa catctccgac accatctctt ccaacgttga cgacaagacc atccacgaga 720
tgtacctttg gcccttcgcc gacgccgtcc gtgccggtgt cggtgccatc atgtgctcct 780
acaaccaaat caacaactcc tacggttgcc aaaactccta cacccttaac aagttgttga 840
aggccgagct tggtttccaa ggtttcgtca tgtccgactg gggtgcccac cactccggtg 900
ttggttccgc ccttgccggt cttgacatgt ccatgcccgg tgacatcacc ttcgactccg 960
ctacctcctt ctggggcacc aaccttacca ttgccgtcct taacggtact gtcccccaat 1020
ggcgtgttga cgacatggcc gtccgtatca tggccgccta ctacaaggtc ggtcgtgacc 1080
gtctttacca accccccaac ttctcctcct ggacccgtga cgagtacggt ttcaagtact 1140
tctaccccca agaggtcccc tacgagaagg ttaaccactt cgtcaacgtc caacgtaacc 1200
actccgaggt catccgtaag ttgggtgccg actccaccgt ccttttgaag aacaacaacg 1260
ccttgccctt gaccggtaag gagcgtaagg tcgccatcct tggtgaggac gccggttcca 1320
actcttacgg tgccaacggt tgctccgacc gtggttgcga caacggtact cttgctatgg 1380
cctggggttc cggtactgcc gagttcccct accttgtcac ccccgagcaa gccatccaag 1440
ccgaggtctt gaagcacaag ggttccgtct acgccatcac cgacaactgg gccttgtccc 1500
aagtcgagac tcttgccaag caagcctctg tctcccttgt tttcgtcaac tccgacgccg 1560
gtgagggtta catctccgtt gacggtaacg agggtgaccg taacaacctt accctttgga 1620
agaacggtga caaccttatc aaggccgctg ccaacaactg caacaacacc atcgtcgtca 1680
tccactccgt cggtcccgtc cttgttgacg agtggtacga ccaccccaac gtcaccgcca 1740
tcctttgggc cggtttgccc ggtcaagagt ccggtaactc ccttgccgac gtcctttacg 1800
gtcgtgtcaa ccccggtgcc aagtccccct tcacctgggg taagacccgt gaggcttacg 1860
gtgactacct tgtccgtgag cttaacaacg gtaacggtgc cccccaagac gacttctccg 1920
agggtgtttt catcgactac cgtggtttcg acaagcgtaa cgagactccc atctacgagt 1980
tcggtcacgg tttgtcctac accaccttca actactccgg tctccacatc caagtcctta 2040
acgcctcctc caacgcccaa gtcgccaccg agactggtgc cgcccctacc ttcggtcaag 2100
tcggtaacgc ctccgactac gtctaccccg agggtcttac ccgtatctcc aagttcatct 2160
acccctggct taactctacc gacttgaagg cttcctccgg tgacccctac tacggtgttg 2220
acaccgccga gcacgtcccc gagggtgcca ccgacggttc cccccaaccc gtccttcccg 2280
ctggtggtgg ttccggtggt aaccctcgtc tttacgacga gcttatccgt gtctccgtca 2340
ccgtcaagaa caccggtcgt gtcgccggtg acgccgtccc ccaactttac gtttcccttg 2400
gtggtcccaa cgagcccaag gtcgtccttc gtaagttcga ccgtcttacc ttgaagccct 2460
ccgaggagac tgtctggacc accaccctta cccgtcgtga cctttccaac tgggacgtcg 2520
ccgcccaaga ctgggtcatc acctcctacc ccaagaaggt ccacgtcggt tcctcttccc 2580
gtcaacttcc ccttcacgcc gcccttccca aggtccaatg ataatctaga gctcctgggc 2640
ctcatgggcc ttccgctcac tgcc 2664

<210> 3
<211> 8515
<212> DNA
<213> Artificial Sequence
<220>
<223> Description of Artificial Sequence: pSL6AaBGL1
<400> 3
   cgtacgattt aaatgcggcc gcttcggctg cggcgagcgg gtatcagctc actcaaaggc 60
   ggtaatacgg ttatccacag aatcagggga taacgcagga aagaacatgt gagcaaaagg 120
   ccagcaaaag gccaggaacc gtaaaaaggc cgcgttgctg gcgtttttcc ataggctccg 180
   cccccctgac gagcatcaca aaaatcgacg ctcaagtcag aggtggcgaa acccgacagg 240
   actataaaga taccaggcgt ttccccctgg aagctccctc gtgcgctctc ctgttccgac 300
   cctgccgctt accggatacc tgtccgcctt tctcccttcg ggaagcgtgg cgctttctca 360
   atgctcacgc tgtaggtatc tcagttcggt gtaggtcgtt cgctccaagc tgggctgtgt 420
   gcacgaaccc cccgttcagc ccgaccgctg cgccttatcc ggtaactatc gtcttgagtc 480
   caacccggta agacacgact tatcgccact ggcagcagcc actggtaaca ggattagcag 540
   agcgaggtat gtaggcggtg ctacagagtt cttgaagtgg tggcctaact acggctacac 600
   tagaaggaca gtatttggta tctgcgctct gctgaagcca gttaccttcg gaaaaagagt 660
   tggtagctct tgatccggca aacaaaccac cgctggtagc ggtggttttt ttgtttgcaa 720
   gcagcagatt acgcgcagaa aaaaaggatc tcaagaagat cctttgatct tttctacggg 780
   gtctgacgct cagtggaacg aaaactcacg ttaagggatt ttggtcatga gcttgcgccg 840
   tcccgtcaag tcagcgtaat gctctgccag tgttacaacc aattaaccaa ttctgattag 900
   aaaaactcat cgagcatcaa atgaaactgc aatttattca tatcaggatt atcaatacca 960
   tatttttgaa aaagccgttt ctgtaatgaa ggagaaaact caccgaggca gttccatagg 1020
   atggcaagat cctggtatcg gtctgcgatt ccgactcgtc caacatcaat acaacctatt 1080
   aatttcccct cgtcaaaaat aaggttatca agtgagaaat caccatgagt gacgactgaa 1140
   tccggtgaga atggcaagag cttatgcatt tctttccaga cttgttcaac aggccagcca 1200
   ttacgctcgt catcaaaatc actcgcatca accaaaccgt tattcattcg tgattgcgcc 1260
   tgagcgaggc gaaatacgcg atcgctgtta aaaggacaat tacaaacagg aatcgaatgc 1320
   aaccggcgca ggaacactgc cagcgcatca acaatatttt cacctgaatc aggatattct 1380
   tctaatacct ggaatgctgt tttcccaggg atcgcagtgg tgagtaacca tgcatcatca 1440
   ggagtacgga taaaatgctt gatggtcgga agaggcataa attccgtcag ccagtttagt 1500
   ctgaccatct catctgtaac atcattggca acgctacctt tgccatgttt cagaaacaac 1560
   tctggcgcat cgggcttccc atacaatcgg tagattgtcg cacctgattg cccgacatta 1620
   tcgcgagccc atttataccc atataaatca gcatccatgt tggaatttaa tcgcggcctc 1680
   gtcgagcaag acgtttcccg ttgaatatgg ctcataacac cccttgtatt actgtttatg 1740
   taagcagaca gttttattgt tcatttaaat gcggccgcgt acggcggctt cgatagcttc 1800
   agcctcctta ggagcattca aaccataacg aaggagaagg gaagcagata aaattgtacc 1860
   aacaggatta acaatgccct tgccagcgat atcgggagcg ctaccgtgaa tgggctcaac 1920
   caaacaatga accttttctt ctgattttcc taccacaccg gaaagggagg cagaaggcaa 1980
   aaggcccaag ctaccaggaa tgacagaagc ctcatctgaa ataatgtcac caaacaagtt 2040
   gtcagtcaaa acaacaccgt taagtgtacg agggctcttg accaaaagca tggctgcgga 2100
   gtcaatgagc tggtttttta aggtaaggtg aggatattcc tccttaaaaa tcttagctac 2160
   agtcttgcgc caaagacgag aagttgccaa aacattagct ttgtcgagta atgtgacggg 2220
   agcaggaggg ttggaagttt cagctaacca agcagccaaa cgagcaatac gagaaacttc 2280
   ttccaaactg taaggccaag tgtccatagc ataacccgat ccgttgtcct cagtgcgctc 2340
   accaaagtaa caacctccag taagttctcg tacaacacaa aaatcgacac cttcaacgat 2400
   ttcaggcttc aaagggctgt acttgactaa agacttgctg gcaaagttgc aaggtcgaag 2460
   gttggcccaa acacccatac tcttacgaag cttcaataaa ccttgctcag gacgacaatt 2520
   ggggttggtc cattcaggac caccaacggc acccaaaaga acaccgtcag cttccaaaca 2580
   agccttcaca gtctcgtcag tcaaaggggt tccataggca tcaatagagg cacctccaat 2640
   cttgtgttct tcaaactcga gttttaactc aggtcgcttc ttctcaacga ctttcaaaac 2700
   ctccaaggca gaagcaacaa tttcagggcc aatatggtct cctggtaaga cgacgatttt 2760
   ctttgcacac atgttgttga agaagttttg ttgtgaaatg gtttcgtgaa agtttcagac 2820
   cctaccgcaa aaatgcctgg tttcgggaaa ctcaacactg ttgcactttt tatactacag 2880
   attgggatat cgataatatt gcgtaaaaaa tccttttttt aaaaagcttg tttacagtaa 2940
   cgtaaatgac cagaaatcag atgaaaatca caagaaagca aataattcac gttaaatcct 3000
   gatatgtttg attttgtgat gaaatcatgg atgttcatag gaattgttga aattgcgctt 3060
   ttttaacgaa atatacaagt atcctggagc ttacttaatt aattaatgaa tctttgttcc 3120
   taggcccggg ctagtaatca attacggggt cattagttca tagcccatat atggagttcc 3180
   gcgttacata acttacggta aatggcccgc ctggctgacc gcccaacgac ccccgcccat 3240
   tgacgtcaat aatgacgtat gttcccatag taacgccaat agggactttc cattgacgtc 3300
   aatgggtgga gtatttacgg taaactgccc acttggcagt acatcaagtg tatcatatgc 3360
   caagtacgcc ccctattgac gtcaatgacg gtaaatggcc cgcctggcat tttgcccagt 3420
   acatgacctt atgggacttt cctacttggc agtacatcta cgtattagtc atcgctatta 3480
   ccatggtgat gcggttttgg cagtacatca atgggcgtgg atagcggttt gactcacggg 3540
   gatttccaag tctccacccc attgacgtca atgggagttt gttttggcac caaaatcaac 3600
   gggactttcc aaaatgtcgt aacaactccg ccccattgac gcaaatgggc ggtaggcgtg 3660
   tacggtggga ggtctatata agcagatttc tctttagttc tttgcaagaa ggtagagata 3720
   aagacacttt ttcaaacatg aagttgtcct ggcttgaggc cgctgccctt accgccgcct 3780
   ccgtcgtttc cgccgacgag ttggccttct cccccccctt ctacccctcc ccctgggcca 3840
   acggtcaagg tgagtgggcc gaggcttacc aacgtgccgt cgccattgtc tcccaaatga 3900
   cccttgacga gaaggtcaac cttaccaccg gtactggttg ggagcttgag aagtgcgtcg 3960
   gtcaaaccgg tggtgtcccc cgtcttaaca tcggtggtat gtgccttcaa gactcccccc 4020
   ttggtatccg tgactccgac tacaactccg ccttccctgc cggtgtcaac gtcgccgcca 4080
   cctgggacaa gaaccttgcc taccttcgtg gtcaagctat gggtcaagag ttctccgaca 4140
   agggtatcga cgtccaactt ggtcctgccg ccggtcccct tggtcgttcc cccgacggtg 4200
   gtcgtaactg ggagggtttc tcccccgacc ccgcccttac cggtgtcctt ttcgccgaga 4260
   ctatcaaggg tatccaagac gctggtgtcg tcgccaccgc caagcactac atccttaacg 4320
   agcaagagca cttccgtcaa gtcgccgagg ccgctggtta cggtttcaac atctccgaca 4380
   ccatctcttc caacgttgac gacaagacca tccacgagat gtacctttgg cccttcgccg 4440
   acgccgtccg tgccggtgtc ggtgccatca tgtgctccta caaccaaatc aacaactcct 4500
   acggttgcca aaactcctac acccttaaca agttgttgaa ggccgagctt ggtttccaag 4560
   gtttcgtcat gtccgactgg ggtgcccacc actccggtgt tggttccgcc cttgccggtc 4620
   ttgacatgtc catgcccggt gacatcacct tcgactccgc tacctccttc tggggcacca 4680
   accttaccat tgccgtcctt aacggtactg tcccccaatg gcgtgttgac gacatggccg 4740
   tccgtatcat ggccgcctac tacaaggtcg gtcgtgaccg tctttaccaa ccccccaact 4800
   tctcctcctg gacccgtgac gagtacggtt tcaagtactt ctacccccaa gagggtccct 4860
   acgagaaggt taaccacttc gtcaacgtcc aacgtaacca ctccgaggtc atccgtaagt 4920
   tgggtgccga ctccaccgtc cttttgaaga acaacaacgc cttgcccttg accggtaagg 4980
   agcgtaaggt cgccatcctt ggtgaggacg ccggttccaa ctcttacggt gccaacggtt 5040
   gctccgaccg tggttgcgac aacggtactc ttgctatggc ctggggttcc ggtactgccg 5100
   agttccccta ccttgtcacc cccgagcaag ccatccaagc cgaggtcttg aagcacaagg 5160
   gttccgtcta cgccatcacc gacaactggg ccttgtccca agtcgagact cttgccaagc 5220
   aagcctctgt ctcccttgtt ttcgtcaact ccgacgccgg tgagggttac atctccgttg 5280
   acggtaacga gggtgaccgt aacaacctta ccctttggaa gaacggtgac aaccttatca 5340
   aggccgctgc caacaactgc aacaacacca tcgtcgtcat ccactccgtc ggtcccgtcc 5400
   ttgttgacga gtggtacgac caccccaacg tcaccgccat cctttgggcc ggtttgcccg 5460
   gtcaagagtc cggtaactcc cttgccgacg tcctttacgg tcgtgtcaac cccggtgcca 5520
   agtccccctt cacctggggt aagacccgtg aggcttacgg tgactacctt gtccgtgagc 5580
   ttaacaacgg taacggtgcc ccccaagacg acttctccga gggtgttttc atcgactacc 5640
   gtggtttcga caagcgtaac gagactccca tctacgagtt cggtcacggt ttgtcctaca 5700
   ccaccttcaa ctactccggt ctccacatcc aagtccttaa cgcctcctcc aacgcccaag 5760
   tcgccaccga gactggtgcc gcccctacct tcggtcaagt cggtaacgcc tccgactacg 5820
   tctaccccga gggtcttacc cgtatctcca agttcatcta cccctggctt aactctaccg 5880
   acttgaaggc ttcctccggt gacccctact acggtgttga caccgccgag cacgtccccg 5940
   agggtgccac cgacggttcc ccccaacccg tccttcccgc tggtggtggt tccggtggta 6000
   accctcgtct ttacgacgag cttatccgtg tctccgtcac cgtcaagaac accggtcgtg 6060
   tcgccggtga cgccgtcccc caactttacg tttcccttgg tggtcccaac gagcccaagg 6120
   tcgtccttcg taagttcgac cgtcttacct tgaagccctc cgaggagact gtctggacca 6180
   ccacccttac ccgtcgtgac ctttccaact gggacgtcgc cgcccaagac tgggtcatca 6240
   cctcctaccc caagaaggtc cacgtcggtt cctcttcccg tcaacttccc cttcacgccg 6300
   cccttcccaa ggtccaatga taatctagag tcgacctgca ggcatgcaag cttaaatagg 6360
   aaagtttctt caacaggatt acagtgtagc tacctacatg ctgaaaaata tagcctttaa 6420
   atcattttta tattataact ctgtataata gagataagtc cattttttaa aaatgttttc 6480
   cccaaaccat aaaaccctat acaagttgtt ctagtaacaa tacatgagaa agatgtctat 6540
   gtagctgaaa ataaaatgac gtcacaagac gatctgcctc gcgcgtttcg gtgatgacgg 6600
   tgaaaacctc tgacacatgc agctcccgga gacggtcaca gcttgtctgt aagcggatgc 6660
   cgggagcaga caagcccgtc agggcgcgtc agcgggtgtt ggcgggtgtc ggggcgcagc 6720
   catgacccag tcacgtagcg atagcggagc ccgggcacta gtgaattcga gtatgtgtac 6780
   gagttgtctt taaacccaca gaggtagaat gtatatataa aattaataag ctaagtgtaa 6840
   tacttaaaaa atacattaat tggaactcgt atcctaccat ttacaatgtt catccaattt 6900
   tttcagattg tactgtaaat agcgtttgaa aacaccaaat tttagaagct aatcactctc 6960
   atcataatcg tctacatcct catcgttatc gacgataaaa gaatcatctt gcatgctggg 7020
   ttcatccatg ctatcaaacg agggatcaac gtaaataggt gttttcactg tagccgctgc 7080
   tcttctggtt ggcctctttc taatcggaga atctgaatct tctggtggct ctgcgttagt 7140
   cgaactagct tttggagttg aactactacc tggaataata aaatcatcat cgtcatcttc 7200
   aggtgattgt ttctttaccg agcttgcttt tttcccttta ttcttcgcag aagccttcgt 7260
   ggatgttatg gtggaaggtt tcaaactgct aggcaacaaa tcatcttcat cgtctgaaga 7320
   aaatatggta gtagcaactg gtttattagt ctttcttcct cttccagacg ccgaggctgc 7380
   tatttttttg acgggttttt tactacctgc gtcttcagag tcaacagatt gacttctttt 7440
   tcttgatttt ccactatcac tgctatccaa tcccgggctc ttagatatgc gattttcttc 7500
   aactgataag ccatgagagt tatcctctgt cttgacaatg tttatgtcag atgatttctc 7560
   aggttctttc gacgctgcga actcaagtaa agtttgttgc tttcgatttg ttgtagatgg 7620
   tttggattcg ctgctagctt cttttttaac agcagtactt gaggaggatc cggcaatagc 7680
   cctgggtttc ctagtaccag tggatttacc tcgaggcttc tttttcgttc gatttacaaa 7740
   atctcttgag gattgctctt cttctaacat ttctctctga atatcatcca taaccttatt 7800
   ccaagcatgc tcaaatgcat ccaaatcatg aagccacaat tctttaggag tttttttaat 7860
   caaagcatcc agttcggcca ttacttcgtc ctttttcttg agaagttcca cataccgttc 7920
   ataggtcaaa gaccataaag gcattgaaag aaggtaattg taggcatctg aatcctcgtc 7980
   ttgcgaaaca tcaccagatt gttcttcttc agcaagagca ttttcaactt ctaaatcaac 8040
   caaatgccct ttctttggtt tactgatagg ttgaaacttc ttttccttca gctccacaat 8100
   gagatccttt ttcttctttt ttgaaactac aagctccccc tctataatca tatgaataaa 8160
   ccgcgcttga tttgaaaatc tatcaaacct tttttccaat tcattaacca tatgctcttt 8220
   acgtctctgg tatgtcctta aacgtacttc gtaaaactcg gtcaaaatat cttcaacact 8280
   gtcatacttc ttgatccgtc cagatgcatc aaaagcaatc atattactcg ttgcttgagt 8340
   acgcgacagt ttaaacttaa cttccaagga ttcatttaat gcttctttca tgccagcttc 8400
   ggtaagcgtg acattaaagt gaacatttcc ttcaccgtga tggctttcat agtccacgat 8460
   gaatttacga attttttccg taccaacaag accagcctcc agatactcct tcatt 8515

<210> 4
<211> 8563
<212> DNA
<213> Artificial Sequence
<220>
<223> Description of Artificial Sequence: pSL6P3AaBGL1
<400> 4
ctctgccagt gttacaacca attaaccaat tctgattaga aaaactcatc gagcatcaaa 60
tgaaactgca atttattcat atcaggatta tcaataccat atttttgaaa aagccgtttc 120
tgtaatgaag gagaaaactc accgaggcag ttccatagga tggcaagatc ctggtatcgg 180
tctgcgattc cgactcgtcc aacatcaata caacctatta atttcccctc gtcaaaaata 240
aggttatcaa gtgagaaatc accatgagtg acgactgaat ccggtgagaa tggcaagagc 300
ttatgcattt ctttccagac ttgttcaaca ggccagccat tacgctcgtc atcaaaatca 360
ctcgcatcaa ccaaaccgtt attcattcgt gattgcgcct gagcgaggcg aaatacgcga 420
tcgctgttaa aaggacaatt acaaacagga atcgaatgca accggcgcag gaacactgcc 480
agcgcatcaa caatattttc acctgaatca ggatattctt ctaatacctg gaatgctgtt 540
ttcccaggga tcgcagtggt gagtaaccat gcatcatcag gagtacggat aaaatgcttg 600
atggtcggaa gaggcataaa ttccgtcagc cagtttagtc tgaccatctc atctgtaaca 660
tcattggcaa cgctaccttt gccatgtttc agaaacaact ctggcgcatc gggcttccca 720
tacaatcggt agattgtcgc acctgattgc ccgacattat cgcgagccca tttataccca 780
tataaatcag catccatgtt ggaatttaat cgcggcctcg tcgagcaaga cgtttcccgt 840
tgaatatggc tcataacacc ccttgtatta ctgtttatgt aagcagacag ttttattgtt 900
catttaaatg cggccgcgta cggcggcttc gatagcttca gcctccttag gagcattcaa 960
accataacga aggagaaggg aagcagataa aattgtacca acaggattaa caatgccctt 1020
gccagcgata tcgggagcgc taccgtgaat gggctcaacc aaacaatgaa ccttttcttc 1080
tgattttcct accacaccgg aaagggaggc agaaggcaaa aggcccaagc taccaggaat 1140
gacagaagcc tcatctgaaa taatgtcacc aaacaagttg tcagtcaaaa caacaccgtt 1200
aagtgtacga gggctcttga ccaaaagcat ggctgcggag tcaatgagct ggttttttaa 1260
ggtaaggtga ggatattcct ccttaaaaat cttagctaca gtcttgcgcc aaagacgaga 1320
agttgccaaa acattagctt tgtcgagtaa tgtgacggga gcaggagggt tggaagtttc 1380
agctaaccaa gcagccaaac gagcaatacg agaaacttct tccaaactgt aaggccaagt 1440
gtccatagca taacccgatc cgttgtcctc agtgcgctca ccaaagtaac aacctccagt 1500
aagttctcgt acaacacaaa aatcgacacc ttcaacgatt tcaggcttca aagggctgta 1560
cttgactaaa gacttgctgg caaagttgca aggtcgaagg ttggcccaaa cacccatact 1620
cttacgaagc ttcaataaac cttgctcagg acgacaattg gggttggtcc attcaggacc 1680
accaacggca cccaaaagaa caccgtcagc ttccaaacaa gccttcacag tctcgtcagt 1740
caaaggggtt ccataggcat caatagaggc acctccaatc ttgtgttctt caaactcgag 1800
ttttaactca ggtcgcttct tctcaacgac tttcaaaacc tccaaggcag aagcaacaat 1860
ttcagggcca atatggtctc ctggtaagac gacgattttc tttgcacaca tgttgttgaa 1920
gaagttttgt tgtgaaatgg tttcgtgaaa gtttcagacc ctaccgcaaa aatgcctggt 1980
ttcgggaaac tcaacactgt tgcacttttt atactacaga ttgggatatc gataatattg 2040
cgtaaaaaat ccttttttta aaaagcttgt ttacagtaac gtaaatgacc agaaatcaga 2100
tgaaaatcac aagaaagcaa ataattcacg ttaaatcctg atatgtttga ttttgtgatg 2160
aaatcatgga tgttcatagg aattgttgaa attgcgcttt tttaacgaaa tatacaagta 2220
tcctggagct tacttaatta attaatgaat ctttgttcct aggcccgggc tagtaatcaa 2280
ttacggggtc attagttcat agcccatata tggagttccg cgttacataa cttacggtaa 2340
atggcccgcc tggctgaccg cccaacgacc cccgcccatt gacgtcaata atgacgtatg 2400
ttcccatagt aacgccaata gggactttcc attgacgtca atgggtggag tatttacggt 2460
aaactgccca cttggcagta catcaagtgt atcatatgcc aagtacgccc cctattgacg 2520
tcaatgacgg taaatggccc gcctggcatt ttgcccagta catgacctta tgggactttc 2580
ctacttggca gtacatctac gtattagtca tcgctattac catggtgatg cggttttggc 2640
agtacatcaa tgggcgtgga tagcggtttg actcacgggg atttccaagt ctccacccca 2700
ttgacgtcaa tgggagtttg ttttggcacc aaaatcaacg ggactttcca aaatgtcgta 2760
acaactccgc cccattgacg caaatgggcg gtaggcgtgt acggtgggag gtctatataa 2820
gcagatttct ctttagttct ttgcaagaag gtagagataa agacactttt tcaaacatga 2880

agatcaccgc tgtcattgcc cttttattct cacttgctgc tgcctcacct attccagttg 2940
ccgatcctgg tgtggtttca gttagcctta agaagcgtgc cgacgagttg gccttctccc 3000
cccccttcta cccctccccc tgggccaacg gtcaaggtga gtgggccgag gcttaccaac 3060
gtgccgtcgc cattgtctcc caaatgaccc ttgacgagaa ggtcaacctt accaccggta 3120
ctggttggga gcttgagaag tgcgtcggtc aaaccggtgg tgtcccccgt cttaacatcg 3180
gtggtatgtg ccttcaagac tccccccttg gtatccgtga ctccgactac aactccgcct 3240
tccctgccgg tgtcaacgtc gccgccacct gggacaagaa ccttgcctac cttcgtggtc 3300
aagctatggg tcaagagttc tccgacaagg gtatcgacgt ccaacttggt cctgccgccg 3360
gtccccttgg tcgttccccc gacggtggtc gtaactggga gggtttctcc cccgaccccg 3420
cccttaccgg tgtccttttc gccgagacta tcaagggtat ccaagacgct ggtgtcgtcg 3480
ccaccgccaa gcactacatc cttaacgagc aagagcactt ccgtcaagtc gccgaggccg 3540
ctggttacgg tttcaacatc tccgacacca tctcttccaa cgttgacgac aagaccatcc 3600
acgagatgta cctttggccc ttcgccgacg ccgtccgtgc cggtgtcggt gccatcatgt 3660
gctcctacaa ccaaatcaac aactcctacg gttgccaaaa ctcctacacc cttaacaagt 3720
tgttgaaggc cgagcttggt ttccaaggtt tcgtcatgtc cgactggggt gcccaccact 3780
ccggtgttgg ttccgccctt gccggtcttg acatgtccat gcccggtgac atcaccttcg 3840
actccgctac ctccttctgg ggcaccaacc ttaccattgc cgtccttaac ggtactgtcc 3900
cccaatggcg tgttgacgac atggccgtcc gtatcatggc cgcctactac aaggtcggtc 3960
gtgaccgtct ttaccaaccc cccaacttct cctcctggac ccgtgacgag tacggtttca 4020
agtacttcta cccccaagag ggtccctacg agaaggttaa ccacttcgtc aacgtccaac 4080
gtaaccactc cgaggtcatc cgtaagttgg gtgccgactc caccgtcctt ttgaagaaca 4140
acaacgcctt gcccttgacc ggtaaggagc gtaaggtcgc catccttggt gaggacgccg 4200
gttccaactc ttacggtgcc aacggttgct ccgaccgtgg ttgcgacaac ggtactcttg 4260
ctatggcctg gggttccggt actgccgagt tcccctacct tgtcaccccc gagcaagcca 4320
tccaagccga ggtcttgaag cacaagggtt ccgtctacgc catcaccgac aactgggcct 4380
tgtcccaagt cgagactctt gccaagcaag cctctgtctc ccttgttttc gtcaactccg 4440
acgccggtga gggttacatc tccgttgacg gtaacgaggg tgaccgtaac aaccttaccc 4500
tttggaagaa cggtgacaac cttatcaagg ccgctgccaa caactgcaac aacaccatcg 4560
tcgtcatcca ctccgtcggt cccgtccttg ttgacgagtg gtacgaccac cccaacgtca 4620
ccgccatcct ttgggccggt ttgcccggtc aagagtccgg taactccctt gccgacgtcc 4680
tttacggtcg tgtcaacccc ggtgccaagt cccccttcac ctggggtaag acccgtgagg 4740
cttacggtga ctaccttgtc cgtgagctta acaacggtaa cggtgccccc caagacgact 4800
tctccgaggg tgttttcatc gactaccgtg gtttcgacaa gcgtaacgag actcccatct 4860
acgagttcgg tcacggtttg tcctacacca ccttcaacta ctccggtctc cacatccaag 4920
tccttaacgc ctcctccaac gcccaagtcg ccaccgagac tggtgccgcc cctaccttcg 4980
gtcaagtcgg taacgcctcc gactacgtct accccgaggg tcttacccgt atctccaagt 5040
tcatctaccc ctggcttaac tctaccgact tgaaggcttc ctccggtgac ccctactacg 5100
gtgttgacac cgccgagcac gtccccgagg gtgccaccga cggttccccc caacccgtcc 5160
ttcccgctgg tggtggttcc ggtggtaacc ctcgtcttta cgacgagctt atccgtgtct 5220
ccgtcaccgt caagaacacc ggtcgtgtcg ccggtgacgc cgtcccccaa ctttacgttt 5280
cccttggtgg tcccaacgag cccaaggtcg tccttcgtaa gttcgaccgt cttaccttga 5340
agccctccga ggagactgtc tggaccacca cccttacccg tcgtgacctt tccaactggg 5400
acgtcgccgc ccaagactgg gtcatcacct cctaccccaa gaaggtccac gtcggttcct 5460
cttcccgtca acttcccctt cacgccgccc ttcccaaggt ccaatgataa tctagagtcg 5520
acctgcaggc atgcaagctt aaataggaaa gtttcttcaa caggattaca gtgtagctac 5580
ctacatgctg aaaaatatag cctttaaatc atttttatat tataactctg tataatagag 5640
ataagtccat tttttaaaaa tgttttcccc aaaccataaa accctataca agttgttcta 5700
gtaacaatac atgagaaaga tgtctatgta gctgaaaata aaatgacgtc acaagacgat 5760
ctgcctcgcg cgtttcggtg atgacggtga aaacctctga cacatgcagc tcccggagac 5820
ggtcacagct tgtctgtaag cggatgccgg gagcagacaa gcccgtcagg gcgcgtcagc 5880
gggtgttggc gggtgtcggg gcgcagccat gacccagtca cgtagcgata gcggagcccg 5940
ggcactagtg aattcgagta tgtgtacgag ttgtctttaa acccacagag gtagaatgta 6000
tatataaaat taataagcta agtgtaatac ttaaaaaata cattaattgg aactcgtatc 6060
ctaccattta caatgttcat ccaatttttt cagattgtac tgtaaatagc gtttgaaaac 6120
accaaatttt agaagctaat cactctcatc ataatcgtct acatcctcat cgttatcgac 6180
gataaaagaa tcatcttgca tgctgggttc atccatgcta tcaaacgagg gatcaacgta 6240
aataggtgtt ttcactgtag ccgctgctct tctggttggc ctctttctaa tcggagaatc 6300
tgaatcttct ggtggctctg cgttagtcga actagctttt ggagttgaac tactacctgg 6360
aataataaaa tcatcatcgt catcttcagg tgattgtttc tttaccgagc ttgctttttt 6420
ccctttattc ttcgcagaag ccttcgtgga tgttatggtg gaaggtttca aactgctagg 6480
caacaaatca tcttcatcgt ctgaagaaaa tatggtagta gcaactggtt tattagtctt 6540
tcttcctctt ccagacgccg aggctgctat ttttttgacg ggttttttac tacctgcgtc 6600
ttcagagtca acagattgac ttctttttct tgattttcca ctatcactgc tatccaatcc 6660

cgggctctta gatatgcgat tttcttcaac tgataagcca tgagagttat cctctgtctt 6720
gacaatgttt atgtcagatg atttctcagg ttctttcgac gctgcgaact caagtaaagt 6780
ttgttgcttt cgatttgttg tagatggttt ggattcgctg ctagcttctt ttttaacagc 6840
agtacttgag gaggatccgg caatagccct gggtttccta gtaccagtgg atttacctcg 6900
aggcttcttt ttcgttcgat ttacaaaatc tcttgaggat tgctcttctt ctaacatttc 6960
tctctgaata tcatccataa ccttattcca agcatgctca aatgcatcca aatcatgaag 7020
ccacaattct ttaggagttt ttttaatcaa agcatccagt tcggccatta cttcgtcctt 7080
tttcttgaga agttccacat accgttcata ggtcaaagac cataaaggca ttgaaagaag 7140
gtaattgtag gcatctgaat cctcgtcttg cgaaacatca ccagattgtt cttcttcagc 7200
aagagcattt tcaacttcta aatcaaccaa atgccctttc tttggtttac tgataggttg 7260
aaacttcttt tccttcagct ccacaatgag atcctttttc ttcttttttg aaactacaag 7320
ctccccctct ataatcatat gaataaaccg cgcttgattt gaaaatctat caaacctttt 7380
ttccaattca ttaaccatat gctctttacg tctctggtat gtccttaaac gtacttcgta 7440
aaactcggtc aaaatatctt caacactgtc atacttcttg atccgtccag atgcatcaaa 7500
agcaatcata ttactcgttg cttgagtacg cgacagttta aacttaactt ccaaggattc 7560
atttaatgct tctttcatgc cagcttcggt aagcgtgaca ttaaagtgaa catttccttc 7620
accgtgatgg ctttcatagt ccacgatgaa tttacgaatt ttttccgtac caacaagacc 7680
agcctccaga tactccttca ttcgtacgat ttaaatgcgg ccgcttcggc tgcggcgagc 7740
gggtatcagc tcactcaaag gcggtaatac ggttatccac agaatcaggg gataacgcag 7800
gaaagaacat gtgagcaaaa ggccagcaaa aggccaggaa ccgtaaaaag gccgcgttgc 7860
tggcgttttt ccataggctc cgcccccctg acgagcatca caaaaatcga cgctcaagtc 7920
agaggtggcg aaacccgaca ggactataaa gataccaggc gtttccccct ggaagctccc 7980
tcgtgcgctc tcctgttccg accctgccgc ttaccggata cctgtccgcc tttctccctt 8040
cgggaagcgt ggcgctttct caatgctcac gctgtaggta tctcagttcg gtgtaggtcg 8100
ttcgctccaa gctgggctgt gtgcacgaac cccccgttca gcccgaccgc tgcgccttat 8160
ccggtaacta tcgtcttgag tccaacccgg taagacacga cttatcgcca ctggcagcag 8220
ccactggtaa caggattagc agagcgaggt atgtaggcgg tgctacagag ttcttgaagt 8280
ggtggcctaa ctacggctac actagaagga cagtatttgg tatctgcgct ctgctgaagc 8340
cagttacctt cggaaaaaga gttggtagct cttgatccgg caaacaaacc accgctggta 8400
gcggtggttt ttttgtttgc aagcagcaga ttacgcgcag aaaaaaagga tctcaagaag 8460
atcctttgat cttttctacg gggtctgacg ctcagtggaa cgaaaactca cgttaaggga 8520
ttttggtcat gagcttgcgc cgtcccgtca agtcagcgta atg 8563

## Description

### TECHNICAL FIELD

The present invention relates to a transformant of a yeast of the genus *Schizosaccharomyces,* and a method for producing the transformant. Specifically, the present invention relates to a transformant of a yeast of the genus *Schizosaccharomyces* which can produce *β*-glucosidase, and a method for producing such a transformant.

### BACKGROUND ART

To produce biomass fuels from a cellulosic biomass such as a wood, rice straw, rice husk and weed including a sugar as a fermentation feedstock, and a bioethanol, etc., it is required to decompose the main structural component of a plant cell wall, cellulose. To decompose cellulose, an acid saccharification method such as a concentrated sulfuric acid saccharification method or a dilute sulfuric acid saccharification method, an enzymatic saccharification method, etc. may be employed. Because of recent development in biotechnology, research and development of an enzymatic saccharification method are actively carried out. In the enzymatic saccharification of cellulose, a group of enzymes generally known as cellulases are utilized. Firstly, an endoglucanase (EG), which has an activity to cleave cellulose chains at random, decomposes an amorphous region of cellulose to expose terminal glucose residues. The exposed glucose residues are decomposed by a cellobiohydrolase (CBH) to release cellobiose. Thereafter, the released cellobiose is decomposed by *β*-glucosidase (BGL) to release glucose.

For the saccharification of cellulose, filamentous fungi of the genus *Aspergillus* and the genus *Trichoderma* are widely used, since they can produce various cellulases and hemicellulases which are required for decomposing and saccharifying a crystalline cellulose, and they can secrete a large amount of such enzymes to their extracellular environment.

Further, it has been tried to express such cellulases of filamentous fungi in a heterologous microorganism. Non-patent document 1 discloses that a budding yeast *Saccharomyces cerevisiae* was transformed with a gene encoding *β*-glucosidase 1 (BGL 1) of *Aspergillus aculeatus* to obtain a transformant, and the obtained transformant expressed such an enzyme.

### PRIOR ART DOCUMENT

### NON-PATENT DOCUMENT

Non-Patent Document 1: G. Tanaka, et al., Biosci. Biotechnol. Biochem., 62(8), 1615-1618, 1998.

### DISCLOSURE OF INVENTION

### TECHNICAL PROBLEM

However, in the enzymatic saccharification method, as the enzymatic hydrolysis of cellulose proceeds, glucose accumulates in the reaction system and the accumulated glucose inhibits *β*-glucosidase, whereby accumulation of cellobiose proceeds. Further, there is a problem such that the complete decomposition of cellulose may not be achieved since the accumulated cellobiose inhibits endoglucanase and cellobiohydrolase. Accordingly, development of a highly functional *β-*glucosidase has been desired.

On the other hand, the genetic analysis of a yeast of the genus *Schizosaccharomyces* is more advanced than that of a filamentous fungus of the genus *Aspergillus* or the genus *Trichoderma,* and the yeast has a lot of advantages like availability of various useful mutant strains and gene transfer vectors, and its suitability for industrial large-scale production of a protein. However, the yeast of the genus *Schizosaccharomyces* does not have endogenous *β*-glucosidase gene, whereby it cannot utilize cellobiose.

Further, in Non-Patent Document 1, there is no description about an inhibitory effect of glucose to *β-*glucosidase produced by a budding yeast.

Here, the object of the present invention is to provide a transformant of a yeast of the genus *Schizosaccharomyces* which can produce *β*-glucosidase, and a method for producing such a transformant.

### SOLUTION TO PROBLEM

The present inventors have conducted extensive studies to resolve the above-mentioned problems, and as a result, have found that the above-mentioned problems can be resolved by means of a transformant having a structural gene sequence encoding a *β*-glucosidase derived from a filamentous fungus in a chromosome, or alternatively having the sequence as an extrachromosomal gene. The present invention has been accomplished on the basis of such discovery.

The transformant of a yeast of the genus *Schizosaccharomyces* of the present invention and its production method are shown below as [1] to [14].
[1] A transformant of a yeast of the genus *Schizosaccharomyces* characterized by having a structural gene sequence encoding a *β*-glucosidase derived from a filamentous fungus, and a promoter sequence and a terminator sequence for expressing the structural gene in a chromosome, or alternatively by having the sequences as an extrachromosomal gene. [2] The transformant of a yeast of the genus *Schizosaccharomyces* according to [1], wherein the *β*-glucosidase is BGL1. [3] The transformant of a yeast of the genus *Schizosaccharomyces* according to [1] or
[2], wherein the filamentous fungus is a microorganism of the genus *Aspergillus.* [4] The transformant of a yeast of the genus *Schizosaccharomyces* according to any one of [1] to [3], wherein the *β*-glucosidase is comprised of an amino acid sequence of SEQ ID NO: 1, or is comprised of the amino acid sequence having deletion, substitution or addition of at least one amino acid, and has a catalytic activity to hydrolyze a *β*-D-glucopyranoside bond. [5] The transformant of a yeast of the genus *Schizosaccharomyces* according to any one of [1] to [4], which further has a structural gene sequence of a secretion signal capable of functioning in the yeast of the genus *Schizosaccharomyces* at the 5' end side of the structural gene sequence encoding the *β*-glucosidase. [6] A transformation method for a yeast of the genus *Schizosaccharomyces,* characterized in that the yeast of the genus *Schizosaccharomyces* is transformed by using a vector having a structural gene sequence encoding a *β*-glucosidase derived from a filamentous fungus, and a promoter sequence and a terminator sequence for expressing the structural gene. [7] The transformation method for a yeast of the genus *Schizosaccharomyces* according to [6], wherein a structural gene sequence of a secretion signal capable of functioning in the yeast of the genus *Schizosaccharomyces* is located at the 5' end side of the structural gene sequence encoding the *β*-glucosidase. [8] The transformation method for a yeast of the genus *Schizosaccharomyces* according to [6] or [7], wherein the structural gene sequence encoding a *β*-glucosidase derived from a filamentous fungus is integrated into at least one position of a chromosome of the yeast of the genus *Schizosaccharomyces.* [9] The transformation method for a yeast of the genus *Schizosaccharomyces* according to [8], wherein the gene sequence encoding the *β-*glucosidase is integrated into the chromosome by homologous recombination. [10] The transformation method for a yeast of the genus *Schizosaccharomyces* according to [9], wherein the vector is integrated into a transposon gene Tf2 site. [11] A method for producing a *β*-glucosidase, characterized in that the *β*-glucosidase is recovered from cells obtained by incubating the transformant as defined in any one of [1] to [4]. [12] A method for producing a *β*-glucosidase, characterized in that the *β-*glucosidase is recovered from a culture broth obtained by incubating the transformant as defined in [5]. [13] A cellulose decomposition method, characterized in that the *β-*glucosidase obtained by the production method as defined in [11] or [12] is used. [14] A cellulose decomposition method, characterized in that the transformant as defined in [5] is cultivated in the presence of cellulose.

### ADVANTAGEOUS EFFECTS OF INVENTION

According to the present invention, it is possible to provide a transformant of a yeast of the genus *Schizosaccharomyces* which can produce *β*-glucosidase, and a method for producing such a transformant.

Further, the transformant of a yeast of the genus *Schizosaccharomyces* of the present invention can produce a *β*-glucosidase which is highly resistant to glucose inhibition.

### BRIEF DESCRIPTION OF DRAWINGS

[Fig. 1] Fig. 1 is a structure map of the expression vector pSL6AaBGL1.
[Fig. 2] Fig. 2 is a structure map of the expression vector pSL6P3AaBGL1.
[Fig. 3] Fig. 3 is a graph showing the results of the optimal temperature test of Test Example 7, and indicates the activity of a crude *β*-glucosidase obtained in Test Example 6 in the each temperature point.
[Fig. 4] Fig. 4 is a graph showing the results of the optimal pH test of Test Example 7, and indicates the activity of a crude *β*-glucosidase obtained in Test Example 6 in the each pH point.
[Fig. 5] Fig. 5 is a graph showing the results of the thermostability test of Test Example 7, and indicates the activity of a crude *β-*glucosidase obtained in Test Example 6 measured after 10 minutes treatment at a temperature defined in the each temperature point.
[Fig. 6] Fig. 6 is a graph showing the results of the pH stability test of Test Example 7, and indicates the activity of a crude *β*-glucosidase obtained in Test Example 6 measured after treating it by a pH of the each pH point at 37°C for 24 hours.
[Fig. 7] Fig. 7 is a graph showing the results of the cultivation test of Test Example 8, in which the transformants were cultivated in the presence of cellobiose. (A) shows the results obtained when they were cultivated in YPD medium, and (B) shows the results obtained when they were cultivated in YP+cellobiose medium.
[Fig. 8] Fig. 8 is a graph showing the results of the glucose inhibition test of Test Example 9.
[Fig. 9] Fig. 9 is a photograph showing the results of SDS-PAGE of Test Example 10.
[Fig. 10] Fig. 10 is a graph showing the results of the thermostability test of Test Example 10.
[Fig. 11] Fig. 11 is a graph showing the results of the glucose inhibition test of Test Example 10.
[Fig. 12] Fig. 12 is a graph showing the results of the N-linked saccharide chain removal of Test Example 11.
[Fig. 13] Fig. 13 is a graph showing the results of the thermostability test of Test Example 11.
[Fig. 14] Fig. 14 is a graph showing the results of the glucose inhibition test of Test Example 11.
[Fig. 15] Fig. 15 is a graph showing the activity enhancement effect of BGL1 derived from ASP3326 strain of Test Example 12.
[Fig. 16] Fig. 16 is a graph showing the activity enhancement effect of BGL1 derived from ASP3326 strain, ASP3396 strain or ASP3397 strain of Test Example 12.

### DESCRIPTION OF EMBODIMENTS

### [Transformant]

The transformant of the present invention is a transformant of a yeast of the genus *Schizosaccharomyces* characterized by having a structural gene sequence encoding a *β-*glucosidase derived from a filamentous fungus, and a promoter sequence and a terminator sequence for expressing the structural gene in a chromosome, or alternatively by having the sequences as an extrachromosomal gene. (Host)

The host of the transformant of the present invention is a yeast of the genus *Schizosaccharomyces.* The yeast of the genus *Schizosaccharomyces* to be used in the present invention may be a wild-type or a mutant-type in which a specific gene is deleted or inactivated depending on application. For deletion or inactivation of a specific gene, conventional methods can be used. Specifically, the Latour system (Nucleic Acids Res. (2006) 34: e11, and WO2007/063919) can be used to delete the gene. Further, the gene can be inactivated by mutating the gene at a certain position by mutant screening using mutagens (Koubo Bunshi Idengaku Jikken-Hou, 1996, Japan Scientific Societies Press), random mutations using PCR (polymerase chain reaction) (PCR Methods Appl., 1992, vol. 2, p.28-33) and the like. As the yeast of the genus *Schizosaccharomyces* host in which a specific gene is deleted or inactivated, ones disclosed in WO2002/101038, WO2007/015470, etc. may, for example, be used.

Further, it is preferred to use a yeast of the genus *Schizosaccharomyces* host having a marker for selecting a transformant. For example, it is preferred to use a host which essentially requires a specific nutrient factor for growth due to deletion of a gene. By using a vector carrying the deleted gene (auxotrophic complementation marker), a transformant lacking the auxotrophy of the host will be obtained. It is possible to select the transformant by using the difference in auxotrophy between the host and the transformant.

For example, a yeast of the genus *Schizosaccharomyces* host which has been made auxotrophic for uracil by deletion or inactivation of orotidine 5'-phosphate decarboxylase (ura4 gene) is transformed with a vector containing ura4 gene (auxotrophic complementation marker), and transformants carrying the vector are obtained by selecting ones lacking uracil auxotrophy. The gene to be deleted to make an auxotrophic host is not limited to ura4 gene when it is used for selection of a transformant, and may, for example, be isopropyl malate dehydrogenase gene (leu1 gene).

As the yeast of the genus *Schizosaccharomyces, Schizosaccharomyces pombe, Schizosaccharomyces japonicus,* and *Schizosaccharomyces octosporus* may, for example, be mentioned. Among the above-mentioned yeasts of the genus *Schizosaccharomyces, Schizosaccharomyces pombe* is preferred in view of the availability of various useful mutant strains.

### (Transformant)

The transformant of the present invention has an expression cassette containing a structural gene sequence encoding a *β*-glucosidase derived from a filamentous fungus, and a promoter sequence and a terminator sequence for expressing the structural gene in a chromosome, or alternatively, as an extrachromosomal gene. Here, having the above-described expression cassette in a chromosome means that the expression cassette is integrated into at least one position on the chromosome of the yeast of the genus *Schizosaccharomyces,* and having as an extrachromosomal gene means that a plasmid having the expression cassette is contained in the yeast cell. From the viewpoint of easiness in subculture passage of the transformant, it is preferred to have the expression cassette in a chromosome.

Further, the expression cassette is a combination of DNA essential for expressing *β*-glucosidase, and contains a structural gene sequence encoding *β*-glucosidase, and a promoter and a terminator capable of functioning in a yeast of the genus *Schizosaccharomyces.*

The expression cassette may additionally contain at least one of a 5'-untranslated region and a 3'-untranslated region. The expression cassette preferably contains all of the structural gene sequence encoding a *β*-glucosidase derived from a filamentous fungus, the promoter, the terminator, the 5'-untranslated region and the 3'-untranslated region. Further, genes such as an auxotrophic complementation marker may be contained.

Further, since recovery and purification of *β*-glucosidase become easier when the amount of *β*-glucosidase secreted out of the cells of a yeast of the genus *Schizosaccharomyces* is large, it is preferred that a nucleotide sequence encoding a secretion signal sequence (secretion signal structural gene) capable of functioning in a yeast of the genus *Schizosaccharomyces* is located at the 5' end side of the structural gene encoding *β*-glucosidase. The 5' end side of the structural gene encoding *β-*glucosidase is an upstream region of the structural gene encoding *β*-glucosidase, and is a position adjacent to the 5' end of the structural gene encoding *β*-glucosidase. Further, a nucleotide sequence encoding a number of amino acids in the N-terminal side, which does not affect the activity of *β*-glucosidase, may be removed and a gene encoding a secretion signal sequence may be introduced thereto.

The promoter and the terminator may be ones capable of functioning in a yeast of the genus *Schizosaccharomyces* host to direct expression of a *β*-glucosidase derived from a filamentous fungus. As the promoter capable of functioning in a yeast of the genus *Schizosaccharomyces,* a promoter intrinsic to the yeast of the genus *Schizosaccharomyces* (preferably one having a high transcriptional activity) or a promoter extrinsic to the yeast of the genus *Schizosaccharomyces* (such as a promoter derived from a virus) may be used. Further, two or more types of promoters may be contained in the vector.

As the promoter intrinsic to the yeast of the genus *Schizosaccharomyces,* a promoter of alcohol dehydrogenase gene, a promoter of nmt1 gene which relates to thiamine metabolism, a promoter of fructose 1,6-bisphosphatase gene which relates to glucose metabolism, a promoter of an invertase gene which relates to catabolite repression (WO99/23223) or a promoter of a heat shock protein gene (WO2007/26617) may, for example, be mentioned.

As the promoter extrinsic to the yeast of the genus *Schizosaccharomyces,* a promoter derived from an animal cell virus disclosed in JP-A-5-15380, JP-A-7-163373 or JP-A-10-234375 may, for example, be mentioned, and hCMV promoter or SV40 promoter is preferred.

As the terminator capable of functioning in a yeast of the genus *Schizosaccharomyces,* a terminator intrinsic to the yeast of the genus *Schizosaccharomyces,* or a terminator extrinsic to the yeast of the genus *Schizosaccharomyces,* may be used. Further, two or more types of terminators may be contained in the vector.

As the terminator, the terminator derived from the human disclosed in JP-A-5-15380, JP-A-7-163373 or JP-A-10-234375 may, for example, be mentioned, and human lipocortin-I terminator is preferred.

### (β-glucosidase)

*β*-glucosidase (EC.3.2.1.21) is a generic name of an enzyme which specifically catalyzes hydrolysis of a *β*-D-glucopyranoside bond. Particularly, it is also called as cellobiase since it decomposes cellobiose to glucose, and is widely found in bacteria, filamentous fungi, plants and animals. A plurality of genes encoding *β*-glucosidase is usually found in each of the species, and for example, existence of bgl1 to bgl7 in a filamentous fungus *Aspergillus oryzae* has been reported (Soy Protein Research, Japan, Vol. 12, pp. 78-83, 2009; and JP-A-2008-086310). Among them, bgl1 which encodes BGL1 is preferred from the viewpoint of its high activity, etc.

The structural gene encoding *β*-glucosidase contained in the transformant of the present invention is derived from a filamentous fungus. The filamentous fungus is, among fungi, an eukaryotic microorganism composed of tubular cells called hyphae. As the filamentous fungus, a fungus of the genus *Aspergillus,* the genus *Trichoderma,* the genus *Fusarium,* the genus *Penecillum* or the like may be mentioned. The *β-*glucosidase structural gene of the present invention may be derived from any filamentous fungus so long as it produces *β*-glucosidase, but is preferably a *β-*glucosidase derived from a filamentous fungus of the genus *Aspergillus* from the viewpoint of its high enzymatic activity, etc. The filamentous fungus of the genus *Aspergillus* may, for example, be *Aspergillus nidulans, Aspergillus oryzae, Aspergillus aculeatus, Aspergillus niger* and *Aspergillus pulverulentus.* The gene encoding a *β-*glucosidase derived from *Aspergillus aculeatus* is preferred since it has high crystalline cellulose decomposition ability and high yield of monosaccharide, and the gene encoding BGL1 (AaBGL1) derived from *Aspergillus aculeatus* is more preferred.

According to the doctoral dissertation of Dr. Reiichiro Sakamoto (Research on cellulase system of *Aspergillus aculeatus* No. F-50, Osaka Prefecture Univerisity, 1984), the wild type AaBGL1 purified from *Aspergillus aculeatus* has a molecular weight of about 133 KDa, an optimal pH of 4.0, and a stable pH range of from 3 to 7 (25°C, 24 hours).

The amino acid sequence of AaBGL1 is an amino acid sequence of SEQ ID No: 1. The gene sequence encoding a *β*-glucosidase of the present invention is preferably a gene sequence encoding a *β*-glucosidase comprised of the amino acid sequence of SEQ ID No: 1. Further, it may be a gene sequence encoding a *β-*glucosidase comprised of the amino acid sequence of SEQ ID No:1 having deletion, substitution or addition of from one to tens amino acids, preferably from one to few amino acids, more preferably from one to nine amino acids, and has a catalytic activity to hydrolyze a *β*-D-glucopyranoside bond. The *β*-glucosidase comprised of the amino acid sequence of SEQ ID No: 1 is one retains a catalytic activity to hydrolyze a *β*-D-glucopyranoside bond even in a case where deletion, substitution or addition of from one to tens amino acids is introduced into the sequence.

The above-described gene encoding a *β*-glucosidase derived from a filamentous fungus may be used as it is. However, to increase expression in yeast of the genus *Schizosaccharomyces,* it is preferred to modify the above-described gene sequence by changing its codons to ones frequently used in a gene highly expressed in yeast of the genus *Schizosaccharomyces.*

### [Transformation Method]

The transformation method of a yeast of the genus *Schizosaccharomyces* of the present invention is characterized in that the yeast of the genus *Schizosaccharomyces* is transformed by using a vector having a structural gene sequence encoding a *β-*glucosidase derived from a filamentous fungus, and a promoter sequence and a terminator sequence for expressing the structural gene.

The yeast of the genus *Schizosaccharomyces* to be used as a host, *β-*glucosidase, filamentous fungus, promoter sequence and terminator sequence are as described above.

### (Vector)

The vector of the present invention contains the above-mentioned expression cassette. Further, a selection marker may preferably be contained in the vector. For example, a vector having an auxotrophic complementation marker proper for the auxotrophy of the host is preferably used.

Further, the vector of the present invention preferably contains a secretion signal gene capable of functioning in yeast of the genus *Schizosaccharomyces.* The secretion signal gene is located at the 5' end side of the structural gene sequence encoding *β*-glucosidase. The secretion signal gene capable of functioning in yeast of the genus *Schizosaccharomyces* is a gene encoding an amino acid sequence having a function of secreting the expressed heterologous protein out of the host cell. A heterologous protein to which the secretion signal is bound at the N-terminal is expressed from a heterologous protein structural gene to which the secretion signal gene is attached. The secretion signal is removed from the protein in the endoplasmic reticulum and the Golgi apparatus, etc. of the host cell, and then, the heterologous protein detached from the secretion signal is secreted out of the host cell. The secretion signal gene (and the secretion signal) should be capable of functioning in yeast of the genus *Schizosaccharomyces.* As the secretion signal gene capable of functioning in yeast of the genus *Schizosaccharomyces,* the secretion signal genes described in WO1996/23890 may be used.

In the present invention, the structural gene of the secretion signal is introduced at the 5' end side of the structural gene sequence encoding *β*-glucosidase, whereby it becomes possible to express *β*-glucosidase to which the secretion signal is bounded at the N-terminal, and then secrete *β*-glucosidase out of the cells of yeast of the genus *Schizosaccharomyces.* As the secretion signal capable of functioning in yeast of the genus *Schizosaccharomyces,* P3 signal described in W01996/23890 is particularly preferred.

The vector of the present invention is a vector having a circular DNA structure or a linear DNA structure. In the case of producing a transformant in which the above-described expression cassette is contained in the cells of yeast of the genus *Schizosaccharomyces* as an extrachromosomal gene, the vector is preferably a plasmid which contains a sequence required for replication in yeast of the genus *Schizosaccharomyces,* i.e. Autonomously Replicating Sequence (ARS).

In the case of producing a transformant in which the above-described expression cassette is integrated into the chromosome of yeast of the genus *Schizosaccharomyces,* the vector is preferably introduced into the host cells in the form of a linear DNA structure.

As described above, a transformant in which the expression cassette is integrated into the chromosome of yeast of the genus *Schizosaccharomyces* is preferred from the viewpoint of subculture passage of the transformant. Here, the vector for producing the transformant in which the expression cassette is integrated into the chromosome of yeast of the genus *Schizosaccharomyces* will be described.

The integration of the expression cassette into the chromosome may be carried out by homologous recombination or non-homologous recombination, but is preferably carried out by homologous recombination since it is possible to integrate the expression cassette into any position of the chromosome by homologous recombination.

When introducing the vector into the host cells in the form of a linear DNA structure, in the case of using a vector having a circular DNA structure like a usual plasmid DNA, it is preferred that the vector is cut open to a linear form by a restriction enzyme before introduction to yeast of the genus *Schizosaccharomyces* cells. In this case, the vector having a circular DNA structure is cut open at a position within the recombination region. The resulting vector has parts of the recombination regions exist at both ends and is integrated entirely into the target site of a chromosome by homologous recombination.

The vector may be constructed by other methods without cutting a vector having a circular DNA structure, for example, by enzymatic amplification by PCR or a chemical synthesis, so long as a linear DNA structure having parts of the recombination region at both ends can be obtained.

To construct the vector of the present invention, a plasmid derived from *E. coli* such as pBR322, pBR325, pUC118, pUC119, pUC18, pUC19 or the like may suitably be used. A vector constructed by using a plasmid derived from *E. coli* usually has the replication origin region called "ori" which is necessary for replication in *E. coli.* Further, even in a case where a plasmid derived from *E*. *coli* like those mentioned above is not used, when *E. coli* is used for construction and amplification of the vector of the present invention, the "ori" is utilized to obtain a vector having "ori".

It is preferred that the replication origin region called "ori" required for replication in *E. coli* is removed from the vector for homologous recombination. Accordingly, it is possible to improve the integration efficiency at the time of integrating the above-described vector into a chromosome (refer to JP-A-2000-262284).

The method for constructing the vector in which the replication origin region is removed is not particularly limited, but is preferably the method disclosed in JP-A-2000-262284. That is, it is preferable to preliminarily construct a precursor vector carrying the replication origin region at a position to be cut within the recombination region so that the replication origin region will be cut-off from the vector at the time of preparing a linear DNA structure. Thus, a vector in which the replication origin region is removed is obtained easily.

Further, it may be a method wherein a precursor vector containing an expression cassette and a recombination region is constructed by using the vectors and their construction methods disclosed in JP-A-5-15380, JP-A-7-163373, WO96/23890, JP-A-10-234375, and then the replication origin region is removed from the precursor vector by using a usual genetic engineering method to obtain a vector to be used for homologous recombination.

The number of copies of the expression cassette in the vector may be only one, or two or more. In a case where the number of copies of the expression cassette in the vector is one, two or more copies of the expression cassette may be integrated in the same position of the chromosome of yeast of the genus *Schizosaccharomyces.* Further, in a case where the number of copies of the expression cassette in the vector is two or more, a plurality of the expression cassettes may be integrated sequentially in the same position of the chromosome of yeast of the genus *Schizosaccharomyces.*

The number of copies of the expression cassette in the vector of the present invention is preferably from 1 to 8, particularly preferably from 2 to 4.

When the number of copies of the expression cassette in the vector is at least 2, it becomes easier to increase the number of copies of the expression cassette integrated into the chromosome of yeast of the genus *Schizosaccharomyces* and expression of a heterologous protein. However, in a case where the number of the after-mentioned target site is large, many copies of the expression cassette can be integrated in the present invention even if the vector has one copy of the expression cassette. Further, when the number of copies of the expression cassette in the vector is at most 8, reduction in the efficiency of vector integration via homologous recombination, which happens in a case where the vector size is too large, is likely to be suppressed. When the number of copies of the expression cassette is at most 4, the vector integration efficiency can be improved further.

The recombination region of the vector is a region having a nucleotide sequence which can induce homologous recombination with a target site in the chromosome of yeast of the genus *Schizosaccharomyces* at which homologous recombination is to be achieved. Further, the target site is a site to become a target for integration of an expression cassette in the chromosome of yeast of the genus *Schizosaccharomyces.* The target site can be designed freely by letting the recombination region of the vector have a nucleotide sequence which induces homologous recombination with the target site.

To induce homologous recombination, the recombination region is required to have a nucleotide sequence with a homology of at least 70% with the target site. Further, the nucleotide sequence homology between the recombination region and the target site is preferably at least 90%, more preferably at least 95%, in view of increasing the efficiency of homologous recombination. By using a vector having such a recombination region, the expression cassette is integrated into the target site by homologous recombination.

The length of the recombination region is preferably from 20 to 2,000 bp. When the length of the recombination region is at least 20 bp, homologous recombination is likely to be induced. Further, when the length of the recombination region is at most 2,000 bp, reduction in the homologous recombination efficiency due to too large vector size is likely to be prevented. The length of the recombination region is preferably at least 100 bp, more preferably at least 200 bp. Further, the length of the recombination region is preferably at most 800 bp, more preferably at most 400 bp.

### (Target site of host)

The target site for integration of the expression cassette is preferably a plurality of target sites in all chromosomes of yeast of the genus *Schizosaccharomyces,* which satisfies either one of conditions (1) two or more target sites exist in different chromosomes or (2) two or more target sites exists at a plurality of positions in the same chromosome separated by at least one essential gene, or both of (1) and (2). These two or more target sites have substantially the same nucleotide sequence. Here, "substantially the same nucleotide sequence" means that there is at least 90% nucleotide sequence homology between target sites. The nucleotide sequence homology between target sites is preferably at least 95%, more preferably at least 99%.

The above-described essential gene is a gene whose lost or inactivation leads to inviability, i.e. a gene essential for growth of the transformant of the present invention. Therefore, when the transformant loses the introduced expression cassettes which are separated by the essential gene, the essential gene is also lost, whereby the transformant cannot grow. Accordingly, during cultivation, the transformant which lost the expression cassettes may not grow along with the transformant retaining the expression cassettes, whereby the heterologous protein production efficiency is unlikely to be reduced.

Because the growth rate of the transformant which lost expression cassettes has a higher growth rate in general, it is preferred to introduce the expression cassettes into target sites which satisfy the above condition (2).

As described above, since the target sites are dispersed in the chromosomes of yeast of the genus *Schizosaccharomyces,* the expression cassettes are integrated into the chromosome in a dispersed manner, whereby the integrated expression cassettes are unlikely to be lost and their passage stability is quite high. Accordingly, it is possible to produce a heterologous protein stably with high productivity.

Further, by designing the target site so as to have substantially the same nucleotide sequence, even in a case where a plurality of target sites exists in different positions, it becomes possible to easily integrate the vector into the respective target sites by using a single vector.

In the transforming method of the present invention, the number of target site positions where the vector is to be integrated is preferably at least 5. When the number of target site positions is at least 5, it is easier to increase the number of copies of the expression cassette integrated into the chromosome, whereby the productivity of a heterologous protein increases further.

Further, the number of target site positions is preferably from 10 to 60. When the number of target site positions is at least 10, it is easier to further increase the number of copies of the expression cassette integrated into the chromosome, whereby the productivity of a heterologous protein increases further. When the number of target site positions is at most 60, reduction in expression of a heterologous protein due to integration of too many copies of the expression cassettes into the chromosome is likely to be suppressed.

The target sites preferably have a nucleotide sequence which exists in a transposon gene Tf2, since it becomes possible to integrate the expression cassettes into the target sites dispersed at plural positions in the chromosome of yeast of the genus *Schizosaccharomyces* at a time by using a single vector. Tf2 is a transposon gene which has a length (the number of nucleotide) of about 4,900 bp and exists in the three chromosomes of yeast of the genus *Schizosaccharomyces* at 13 positions in total, with a nucleotide sequence homology of 99.7% (refer to Nathan J. Bowen et al, Genome Res. 2003 13: 1984-1997).

However, the target sites are not limited to the above-described ones. Other than the transposon gene Tf2, the target sites may, for example, be sites (such as genes) found at plural positions in the chromosomes, and having a length larger than the length of the recombination region and a substantially identical nucleotide sequence. Further, for example, after formation of the target site by newly introducing a plurality of genes (target sites) having a substantially identical nucleotide sequence and a length larger than the length of the recombination region into the chromosomes, the vector may be introduced into a plurality of target sites.

### (Transformation method)

The yeast of the genus *Schizosaccharomyces* host is transformed by using the above-described vector. As the transformation method, any known transformation method for yeast of the genus *Schizosaccharomyces* may be used. Such a transformation method may, for example, be a conventional method like lithium acetate method, electroporation method, spheroplast method, glass-beads method, or the like., and a method disclosed in JP-A-2005-198612. Further, a commercially available yeast transformation kit may be used.

After transformation, the resulting transformants are usually subjected to selection. The selection may, for example, be carried out as follows. Several transformants are selected as viable colonies in a broth via the above-mentioned auxotrophic marker screening method, the transformants are grown separately in a liquid broth, and transformants highly expressing a heterologous protein are selected by measuring expression of a heterologous protein in each culture broth. The number of vectors and copies of the expression cassette integrated into the chromosomes can be identified by analyzing the genomes of the selected transformants by pulse-field gel electrophoresis

### (Cultivation method)

As the culture broth for incubating the transformant of the present invention, a known culture broth for yeasts may be used so long as it contains carbon sources, nitrogen sources, inorganic salts and the like which yeast of the genus *Schizosaccharomyces* can use, and yeast of the genus *Schizosaccharomyces* can grow in it efficiently. The culture broth may be natural or synthetic.

As the carbon sources, saccharides such as glucose, fructose and sucrose may, for example, be mentioned.

As the nitrogen sources, inorganic acids or inorganic ammonium salts such as ammonia, ammonium chloride, and ammonium acetate, peptone and casamino acid may, for example, be mentioned.

As inorganic salts, magnesium phosphate, magnesium sulfate and sodium chloride may, for example, be mentioned.

Cultivation may be carried out by using a known cultivation method for yeasts such as a shaking cultivation, a stirring cultivation or the like.

The cultivation temperature is preferably from 23 to 37°C. Further, the cultivation time may be set appropriately.

Cultivation may be carried out batch-wise or continuously.

When the transformant is cultivated to isolate *β*-glucosidase from culture broth or cells, a known protein isolation method may be used. For example, the cells are separated from the culture broth after cultivation, and then the separated cells are disrupted to obtain a cell lysate containing *β*-glucosidase, followed by recovery of *β-*glucosidase from the cell lysate by using a known protein isolation method such as salting-out, column chromatography purification or immunoprecipitation.

Further, in the case of using a transformant having a *β*-glucosidase structural gene to which a secretion signal gene is attached, since it secretes *β*-glucosidase into the culture broth, it is possible to recover *β*-glucosidase from the culture broth by using a known protein isolation method. The transformant may be cultivated continuously to produce *β*-glucosidase by repeating recovery of a culture supernatant containing *β-*glucosidase from the culture broth by means of centrifugation or the like after a certain period of cultivation, and re-cultivation of the remained cells after supplementing them with a culture broth.

The culture broth or the cell lysate may directly be contacted with the object to be decomposed, cellulose, without isolating *β*-glucosidase from the culture broth or the cell lysate containing *β*-glucosidase.

### [Cellulose decomposition method]

In the cellulose decomposition method of the present invention, a *β*-glucosidase recovered from the cells or the culture broth obtained by incubating the above-described transformant of yeast of the genus *Schizosaccharomyces* is used. Further, in the case of using a transformant having a *β-*glucosidase structural gene to which a secretion signal gene is attached, the transformant may be cultivated in a culture broth containing cellulose thereby to decompose the cellulose contained in the culture broth by *β*-glucosidase secreted into the culture broth.

The form of cellulose as an object to be decomposed is not particularly limited, and may be purified cellulose or cellulose contained in a biomass such as a wood, rice straw, rice husk and weed.

In the case of decomposing cellulose by *β-*glucosidase secreted out from the transformant cultivated in the presence of cellulose, as the cultivation condition, general cultivation conditions for yeast of the genus *Schizosaccharomyces* may be used. The suitable pH of the culture broth is from 3.0 to 8.0, and the suitable cultivation temperature is from 23 to 37°C . In the case of decomposing cellulose by using an isolated and purified *β*-glucosidase produced from the transformant, the reaction conditions may be known reaction conditions for *β*-glucosidase. The suitable pH for *β*-glucosidase of the decomposition reaction solution is from 3.0 to 8.0, and the suitable reaction temperature is from 20 to 65°C.

As the specific cellulose decomposition method, a method wherein the transformant is cultivated to isolate or purify *β*-glucosidase from the culture broth or the cells, and then the isolated or purified *β*-glucosidase is cultivated along with a biomass containing cellulose, an endoglucanase, and a cellobiohydrolase may, for example, be mentioned. Further, in the case of using a transformant which secretes *β*-glucosidase, a method wherein the transformant is cultivated and then mixed with the above-mentioned biomass for decomposition, and a method wherein the transformant is cultivated in the presence of the above-mentioned biomass may additionally be mentioned.

### EXAMPLES

Now, the present invention will be described in further detail with reference to specific Examples. However, the present invention is by no means restricted to the following Examples.

### [Test Example 1] Construction of an expression vector

A gene sequence was designed based on the peptide sequence of *β*-glucosidase 1 derived from *Aspergillus aculeatus* F-50 (Kawaguchi,T. et.al., "Gene", Vol. 173 (2), pp. 287-288, (1996)) (hereinafter referred to as AaBGL1) by replacing the codons with codons highly expressed in *Schizosaccharomyces pombe* (refer to SEQ ID NO: 2. hereinafter referred to as AaBGL1 gene). The recognition sequences for KpnI and BspHI were added upstream of the initiation codon. The recognition sequences for XbaI and SacI were added downstream of the stop codon. A plasmid containing these sequences (synthesized by Geneart AG, Regensburg, Germany) was digested with restriction enzymes BspHI and Xbal.

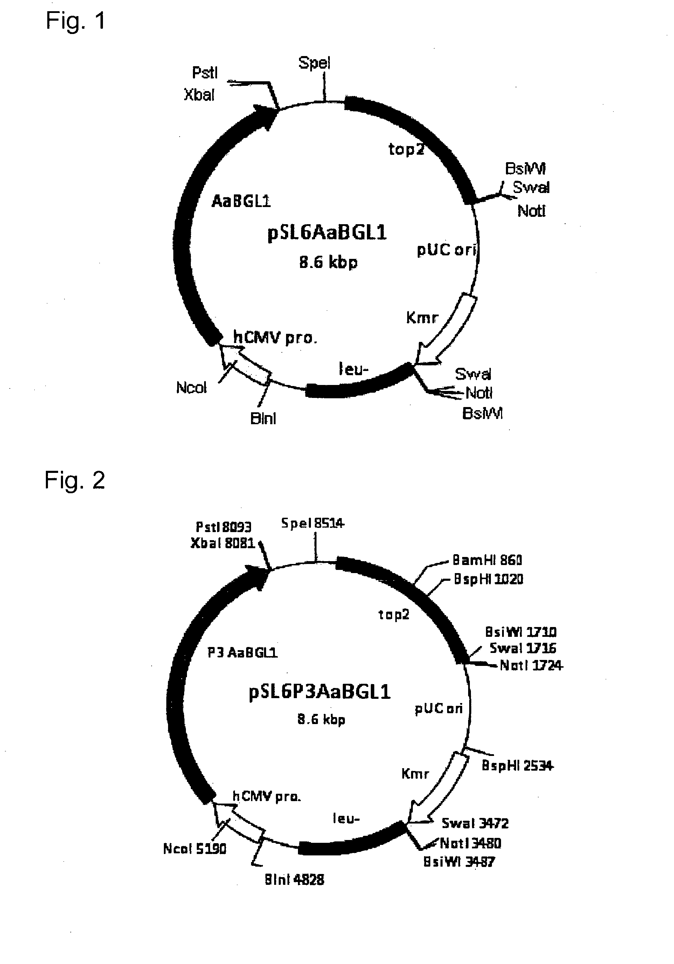

On the other hand, separately therefrom, pSL6lacZ was digested with restriction enzymes Aarl and Xbal, and then treated with an alkaline phosphatase. Thereafter, gel electrophoresis was carried out on an agarose gel to isolate the digested fragment of vector pSL6 and the digested fragment of AaBGL1 gene from the agarose gel, and then these fragments were ligated to each other. The ligated product was introduced into *E*. *coli* DH5α (Takara Bio, Inc.) to obtain a transformant. From the obtained transformant, a vector was prepared to obtain expression vector pSL6AaBGL1 (Fig. 1, refer to SEQ ID NO: 3). The obtained expression vector was confirmed to be a desired vector by restriction enzyme mapping.

Further, to construct AaBGL1 to which secretion signal P3 is bound at the N-terminal, a fragment of AaBGL1 gene was amplified by PCR method with In-fusion primers and pSL6AaBGL1 as template. On the other hand, pSL6P3lacZ was digested with restriction enzymes AfIII and Xbal. The digested fragments and the PCR-amplified product of the AaBGL1 gene fragment were circularized by In-fusion method, and then introduced into *E*. *coli* DH5α (Takara Bio, Inc.) to obtain a transformant. From the obtained transformant, a vector was prepared to obtain expression vector pSL6P3AaBGL1 (Fig. 2, refer to SEQ ID NO: 4). The obtained expression vector was confirmed to be a desired vector by restriction enzyme mapping and partial nucleotide sequencing.

### [Test Example 2] Production of a transformant

As the host cell, a leucine-auxotrophic strain of *Schizosaccharomyces pombe* (genotype: h-, leu1-32, provided from professor Yuichi lino, Molecular genetics research laboratory, Graduate school of science, The university of Tokyo) (ATCC38399) was cultivated in YES medium (0.5% of yeast extract, 3% of glucose and 0.1 mg/ml of SP supplements) until 0.6 x 10⁷ cells/ml. The cells were collected and washed, and then suspended by 0.1M lithium acetate solution (pH 5.0) to 1.0 x 10⁸ cells/ml. Thereafter, to 100 µl of the suspension, 1 µg of the above-mentioned vector pSL6AaBGL1 or pSL6P3AaBGL1 digested by restriction enzyme Notl was added, and then 290 µl of a 50 % (w/v) polyethylene glycol (PEG4000) aqueous solution was added thereto, followed by stirring to cultivate them for 60 minutes at 30 °C, 5 minutes at 42°C, and 10 minutes at room temperature, in this order. PEG4000 was removed by centrifugation and then the cells were washed to suspend them in 150 µl of sterile

water. The suspension was applied on minimal-agarose medium. Three days after cultivation, a transformant (AaBGL1 expression strain) was obtained. One transformed by using pSL6AaBGL1 was designated as ASP3325 strain, and one transformed by using pSL6P3AaBGL1 was designated as ASP3326 strain.

### [Test Example 3] Cultivation of a transformant

TheAaBGL1 expression strain obtained as above was cultivated in YES medium for one day at 30 °C. 100 µl of the culture broth was inoculated on 5 ml of YPD medium (1% of yeast extract, 2% of peptone, and 2% of glucose), followed by cultivation for two days at 30°C. Thereafter, the culture broth was centrifuged to obtain a culture supernatant.

### [Test Example 4] Activity measurement forAaBGL1

By using the above-obtained culture supernatant of the AaBGL1 expression strain, a diluted enzyme sample was prepared, and then the enzymatic activity of the sample was measured in accordance with the following method (activity measurement method).

To 10 µl of 20 mM p-nitrophenyl-*β*-D-glucoside (hereinafter abbreviated as pNPG), 10 µl of 1 M sodium acetate buffer solution (pH 4.5) and 130 µl of water were added, and then 50 µl of the diluted enzyme sample was introduced for reacting them at 37 °C for 10 minutes. 100 µl of the reaction mixture was mixed with 100 µl of 2% sodium carbonate solution to terminate reaction, and then the amount of liberated p-nitrophenol was colorimetrically measured at a wavelength of 450 nm.

The amount of enzyme that produces 1 µmol of p-nitrophenol per minute was defined as 1 U.

As a result, the activity of the diluted enzyme sample derived from ASP3326 strain was found to be 0.85 U/ml, and the activity of the diluted enzyme sample derived from ASP3325 strain was found to be 0.12 U/ml, and accordingly, the activity of the diluted enzyme sample derived from the strain expressing AaBGL1 to which P3 signal is bound (ASP3326 strain) was found to be about seven times higher than the activity derived of the strain expressing AaBGL1 to which P3 signal is not bound (ASP3325 strain).

### [Test Example 5] Fed-batch culture of a strain expressing AaBGL1 to which P3 signal is bound (ASP3326 strain)

ASP3326 strain was inoculated in 100 ml of YES medium, and then subjected to pre-culture at 30°C until OD₆₆₀ reached around 10. Thereafter, by using a jar fermenter, the pre-culture broth was introduced into 1,000 ml of YPD medium, and then cultivated for two days at 30°C while feeding a feed medium having the composition of Table 1 under a condition where glucose depletion did not occur and the glucose concentration did not exceed 1%. The pH was maintained at 4.5 by controlling the addition of 1.5N NaOH solution and 1.5M KOH solution. After completion of the cultivation, centrifugation was carried out to obtain a culture supernatant.

A feeding medium composition

**[Table 1]**

| | |
|---|---|
| Yeast Extract | 100 g |
| Vitamin stock solution (described in the following Table 2) | 1 ml |
| Biotin stock solution (described in the following Table 3) | 100 µl |
| Metal stock solution (described in the following Table 4) | 10 ml |
| Glucose | 500 g |
| CaCl₂ 2H₂O | 0.2 g |
| (NH₄)₂SO₄ | 19.4 g |
| KH₂ PO₄ | 3.9 g |
| MgSO₄ 7H₂O | 1.3 g |
| Na₂H PO₄ | 0.04 g |
| Water | Up to 1,000 ml |

Vitamin stock solution composition

**[Table 2]**

| | |
|---|---|
| Pantothenic acid Ca | 0.01 g |
| Nicotinic acid | 0.10 g |
| Inositol | 0.10 g |
| Water | Up to 10 ml |

Biotin stock solution composition

**[Table 3]**

| | |
|---|---|
| Biotin | 0.001 g |
| Water | Up to 10 ml |

Metal stock solution composition

**[Table 4]**

| | |
|---|---|
| Iron citrate | 1,420 mg |
| ZnSO₄ 7H₂O | 1,270 mg |
| MnCl₂ 4H₂O | 155 mg |
| CuSO₄ 5H₂O | 190 mg |
| H₃BO₃ | 145 mg |
| Na₂MoO₄ 2H₂O | 34 mg |
| Kl | 10 mg |
| NiSO₄ 6H₂O | 22 mg |
| CoCl₂ 6H₂O | 20 mg |
| Water | Up to 1,000 ml |

### [Test Example 6] Purification

Solid ammonium sulfate was introduced into 1,000 ml of the above-obtained culture supernatant until 80% saturation was reached, thereby to salt out impurities. Thereafter, concentration and desalting were carried out by using an ultrafiltration membrane having MWCO of 50,000 and 10 mM acetate buffer solution (pH 5.0) to obtain a crude enzyme.

### [Test Example 7] Characterization analysis

Characterization analysis of the above-obtained crude enzyme was carried out as follows.

### (Optimal temperature)

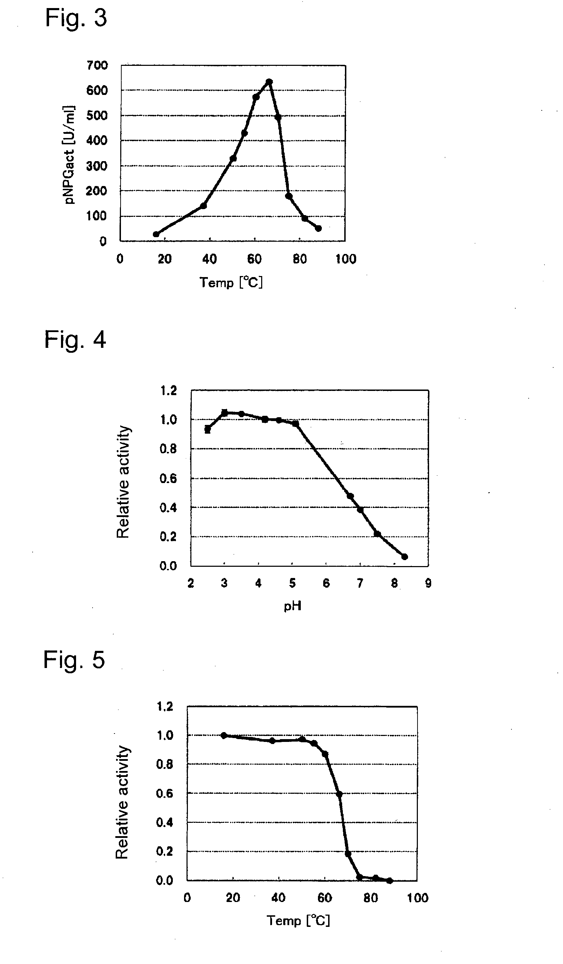

By using 1 mM pNPG as a substrate and 50 mM acetate buffer solution (pH 4.5), the enzyme was reacted for 10 minutes at each temperature. As a result, the optimal temperature for the enzyme was found to be about 65°C (Fig. 3).

### (Optimal pH)

By using 1 mM pNPG as a substrate and various buffer solutions (50 mM glycine-HCl buffer solution, 50 mM acetate buffer solution, 50 mM phosphate buffer solution), the enzyme was reacted for 10 minutes at 37°C under each pH condition. As a result, the optimal pH condition for the enzyme was found to be from pH 3.0 to 5.0 (Fig. 4).

### (Thermostability)

The enzyme was treated at each temperature for 10 minutes, and then reacted by using 1 mM pNPG as a substrate and 50 mM acetate buffer solution (pH 4.5). As a result, the enzyme was found to be stable until about 60°C (Fig. 5).

### (pH stability)

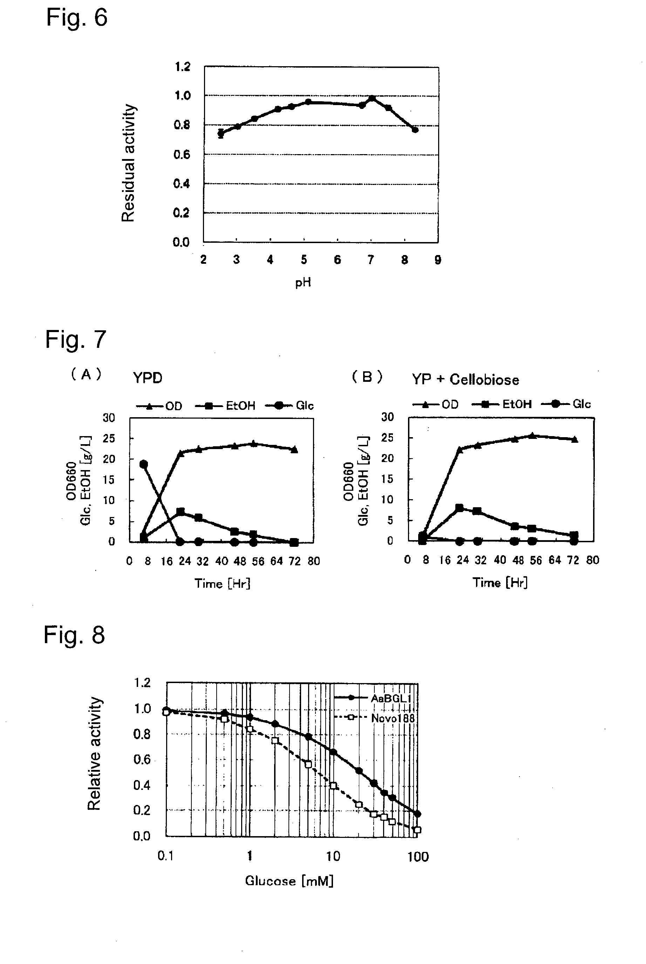

The enzyme was treated under each pH condition for 24 hours at 37°C with various buffer solutions (50 mM glycine-HCl buffer solution, 50 mM acetate buffer solution, 50 mM phosphate buffer solution), and then reacted by using 1 mM pNPG as a substrate. As a result, the enzyme was found to be stable in the vicinity of pH 3.0 to 8.0 (Fig. 6).

### (Molecular weight)

Molecular weight was measured by SDS-PAGE. BenchMark (registered trademark) Protein Ladder manufactured by Invitrogen Inc. was used as a molecular weight marker. As a result, the molecular of the enzyme was found to be about 220,000.

### (Inhibition by various sugar)

By using 1 mM pNPG containing various sugar (0.1 mM, 1.0 mM, 10 mM) as a substrate, the enzyme was reacted in accordance with the activity measurement method. The results are shown in Table 5. The numerical values shown in Table 5 indicate the ratio (%) of the measured enzymatic activity to the enzymatic activity under a condition where no sugar is contained.

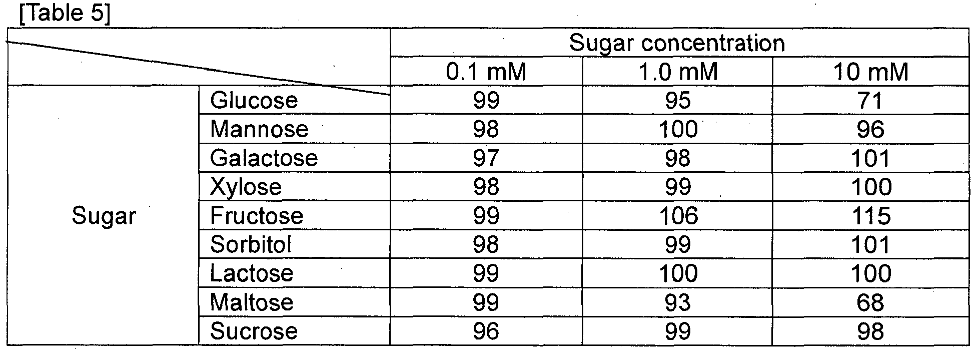

As apparent from Table 5, the above-obtained crude AaBGL1 was not substantially inhibited by mannose, galactose, xylose, fructose, sorbitol, lactose and sucrose, and was inhibited by glucose and maltose.

### (Substrate specificity)

Measured in accordance with the activity measurement method by using various substrates. The specific activities and relative activities of the enzyme to each of 0.5% (w/v) ICOS, 0.5% (w/v) cellobiose, 1 mM pNPG, and 1 mM pNP-cellobiose are shown in Table 6.

### (The activity measurement method using pNP-cellobiose as a substrate)

To 100 µl of 2 mM p-nitrophenyl-*β*-D-cellobioside (hereinafter referred to as pNP-cellobiose), 10 µl of 1 M sodium acetate buffer solution (pH 4.5) and 40 µl of water were added, followed by addition of 50 µl of the diluted enzyme sample to cultivate for 10 minutes at 37°C. 100 µl of the reaction mixture was mixed with 100 µl of 2% sodium carbonate solution to terminate reaction, and then the amount of liberated p-nitrophenol was colorimetrically measured at a wavelength of 450 nm.

The amount of enzyme that produces 1 µmol of p-nitrophenol per minute was defined as 1 U.

### (The activity measurement method using cellobiose as a substrate)

To 100 µl of 1% cellobiose solution, 10 µl of 1 M sodium acetate buffer solution (pH 4.5) was added, followed by addition of 90 µl of the diluted enzyme sample to cultivate for 10 minutes at 37°C. 50 µl of 1 N HCl was added to terminate reaction, and then 50 µl of a neutralizing solution comprised of 1 M Tris solution:2N sodium hydroxide solution in a ratio of 4:1 was added for neutralization to measure the amount of glucose in the mixture by using glucostat method (Glucose kit, Glucose CII-Test Wako, manufactured by Wako Pure Chemical Industries).

The amount of enzyme that produces 1 µmol of glucose per minute was defined as 1 U.

### (Activity measurement method using ICOS as a substrate)

To 100 µl of 1% insoluble cellooligosaccharide (hereinafter referred to as ICOS) solution, 10 µl of 1 M sodium acetate buffer solution (pH 4.5) was added, followed by addition of 90 µl of the diluted enzyme sample to cultivate for 10 minutes at 37°C. 50 µl of 1 N HCl was added to terminate reaction, and then 50 µl of a neutralizing solution comprised of 1 M Tris solution:2N sodium hydroxide solution in a ratio of 4:1 was added for neutralization to measure the amount of glucose in the mixture by using glucostat method (Glucose kit, Glucose CII-Test Wako, manufactured by Wako Pure Chemical Industries).

The amount of enzyme that produces 1 µmol of glucose per minute was defined as 1 U.

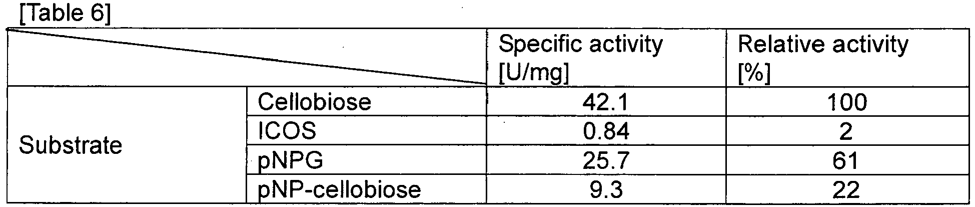

### [Test Example 8] Cultivation in the presence of cellobiose

The strain expressing AaBGL1 to which P3 signal is bound (ASP3326 strain) was cultivated for 3 days at 30°C by using 5 ml of YPD medium (1% of yeast extract, 2% of peptone, and 2% of glucose) and 5 ml of YP+cellobiose medium (1% of yeast extract, 2% of peptone, and 2% of cellobiose).

OD₆₆₀, the glucose concentration and the ethanol concentration of the culture broth were measured. As a result, it was confirmed that the growth curve was equivalent to one obtained in a case where cultivation was carried out in the presence of glucose, and that cellobiose was fermented to ethanol without accumulating glucose.

Fig. 7 (A) shows the results obtained in YPD medium, and Fig. 7 (B) shows the results obtained in YP+cellobiose medium. OD, EtOH and Glc indicate OD₆₆₀ of the culture broth, the ethanol concentration of the culture broth, and the glucose concentration of the culture broth, respectively.

### [Test Example 9] Comparison of glucose inhibition with the case of using Novozyme 188

With regard to glucose inhibition, the crude enzyme obtained in Test Example 6 was compared with a commercially available *β*-glucosidase Novozyme 188 (manufactured by Novozymes). The glucose inhibition activity was measured in accordance with the activity measurement method by using 1 mM pNPG as a substrate and adding glucose to the reaction mixture in a concentration of from 0 to 100 mM. The reaction temperature was 37°C, the pH was 4.5, and the reaction time was 10 minutes. The other reaction conditions were the same as in the activity measurement method described in Test Example 4. The results are shown in Fig. 8.

As apparent from Fig. 8, AaBGL1 obtained in Test Example 6 was more resistant to glucose inhibition than Novozyme 188, and AaBGL1 obtained in Test Example 6 maintained its activity of at least 50% even in a case where 20 mM of glucose was added. The glucose concentration (I₅₀ value) at which 50% inhibition of the enzymatic activity occurs (relative activity of 0.5) was 25 mM for AaBGL1, and was 7 mM for Novozyme 188.

### [Test Example 10] Expression in a glycosylation-deficient strain

*Schizosaccharomyces pombe* strains ARC129 (genotype: h-leu1-32 ura4-C190T pmr1 ::ura4+, provided from professor Kaoru Takegawa, Faculty of Agriculture, Kyushu University) and ARC130 (genotype: h-leu1-32 ura4-C190T pmr1::ura4(FOA) gms1 ::ura4+, provided from professor Kaoru Takegawa, Faculty of Agriculture, Kyushu University) were transformed by using pSL6P3AsBGI1 in accordance with the transformation method described in Test Example 2 to obtain AaBGL1 expression strains. A strain obtained by transforming ARC 129 was designated as ASP3396 strain and a strain obtained by transforming ARC130 was designated as ASP3397 strain.

The above-obtained AaBGL1 expression strains, ASP 3326 strain, and ARC032 strain (which does not express AaBGL1, genotype: h-, provided from professor Yuichi lino, Molecular genetics research laboratory, Graduate school of science, The university of Tokyo) were cultivated in accordance with the cultivation method described in Test Example 3 to obtain culture supernatants. With regard to each of the culture supernatants, the following tests were carried out.

### (SDS-PAGE)

To 1 ml of the culture supernatant, 0.1 ml of trichloroacetic acid was added, followed by cooling on ice for 30 minutes. Thereafter, centrifugation was carried at 15,000 rpm for 20 minutes at 4°C to collect a TCA precipitation sample. After washing, the sample was dissolved in a SDS-PAGE sample buffer solution to carry out SDS-PAGE with a 4 to 12% acrylamide gel, and then stained by Coomassie brilliant blue.

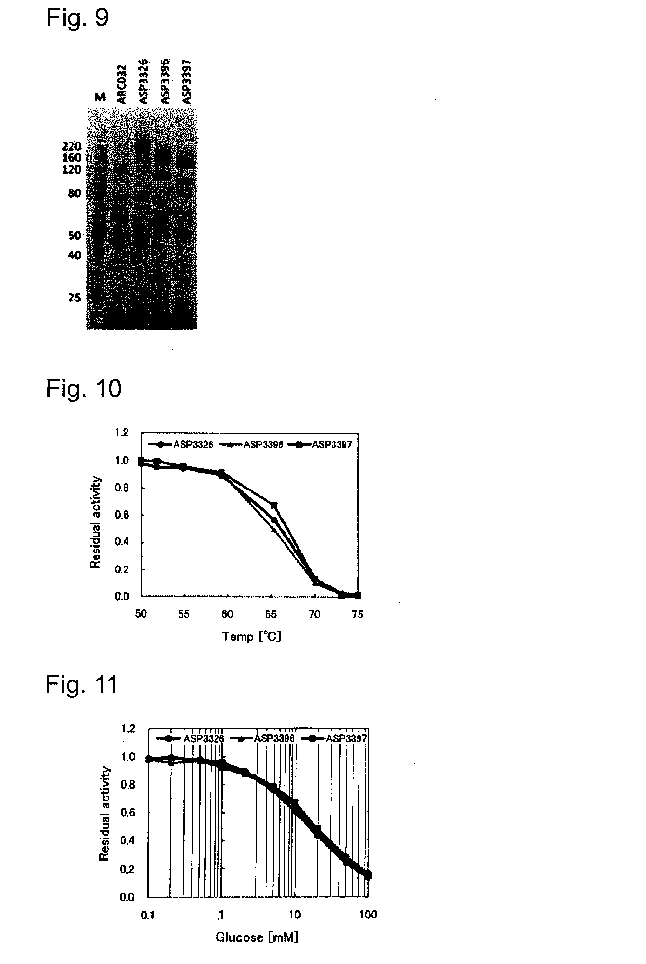

The results are shown in Figure 9. Each of the lanes shown in Figure 9 represents, from left to right, molecular marker (M), a protein of the ARC032 strain culture supernatant, a protein of the ASP3326 strain culture supernatant, a protein of the ASP3396 strain culture supernatant.

As apparent from Fig. 9, the molecular weight of *β*-glucosidase 1 derived from *Aspergillus aculeatus* F-50 was decreased in ASP3396 strain and ASP3397 strain, both derived from saccharide chain-deficient strains, to 160,000 and 130,000, respectively.

### (Activity measurement)

The *β*-glucosidase activity of the culture supernatant obtained from incubating ASP3326 strain, ASP3396 strain or ASP3397 strain was measured in accordance with the activity measurement method described in Test Example 4, by using pNPG as a substrate.

As a result, an activity of 0.85 U/ml for ASP3326 strain, an activity of 1.53 U/ml for ASP3396 strain, an activity of 2.98 U/ml for ASP3397 were observed.

### (Thermostability)

With regard to the each culture supernatant, the thermostability test was carried out in accordance with the thermostability measurement method described in Test Example 7 at a temperature of from 50 to 75°C. The results are shown in Fig. 10.

As apparent from Fig. 10, all the three stains, ASP3326 strain, ASP3396 strain, and ASP3397 strain, were found to be stable until 60°C, and there was no difference by the molecular weight.

### (Glucose inhibition)

With regard to the each culture supernatant, the glucose inhibition activity was measured in accordance with the activity measurement method, by using 1 mM pNPG as a substrate and adding from 0 to 100 mM of glucose to the reaction mixture. The results are shown in Fig. 11.

As apparent from Fig. 11, all the three stains, ASP3326 strain, ASP3396 strain, and ASP3397 strain, showed the similar glucose inhibition activity, and therefore the glucose inhibition activity seemed to be not affected by the existence of a saccharide chain.

### [Test Example 11] Characterization of a purified enzyme after removing N-linked saccharide chain

To each of the culture supernatants obtained by incubating each of ASP3326 strain, ASP3396 strain and ASP3397 strain in the same manner as in Test Example 10, 80% saturation ammonium sulfate was added, and then centrifugation was carried out to collect precipitates. Each of the precipitates was dissolved in 10 mM citrate phosphate buffer solution (pH 7.0), and then equilibrated in 10 mM citrate phosphate buffer solution (pH7.0) by using an ultrafiltration membrane having MWCO of 100,000 (manufactured by Millipore). Thereafter, by using a salt-concentration gradient (0 to 1 M of NaCl) and QXL column (manufactured by GE Healthcare), an anion-exchange chromatography was carried out to collect a fraction of a peak of a *β*-glucosidase activity, thereby to prepare a purified sample.

### (Removal of N-linked saccharide chain)

100 ng of the purified sample was dissolved in a SDS-PAGE sample buffer solution to prepare an untreated sample. On the other hand, 100 ng of the purified sample was subjected to removal of N-linked saccharide chain by using Enzymatic Deglycosylation Kit (Sigma), and then dissolved in a SDS-PAGE sample buffer solution to prepare a N-linked saccharide chain-treated sample. The both samples were subjected to SDS-PAGE with a 4 to 12% acrylamide gel, and then stained by Coomassie brilliant blue.

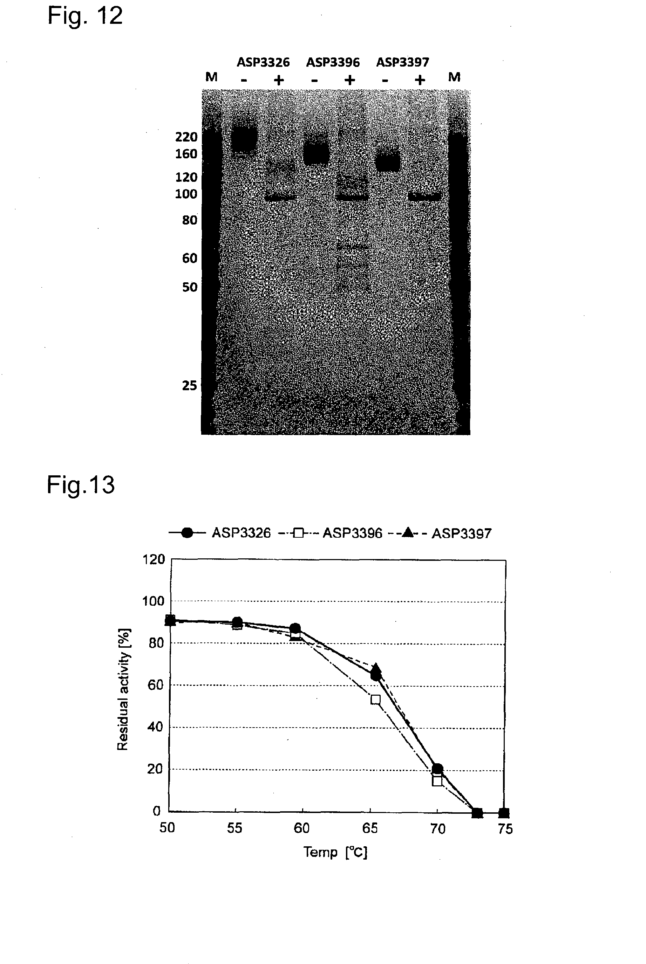

The results are shown in Fig. 12. Each of the lanes shown in Figure 12 represents, from left to right, molecular marker (M), the untreated sample of ARC3326 strain, the N-linked saccharide chain-treated sample of ARC3326 strain, the untreated sample of ARC3396 strain, the N-linked saccharide chain-treated sample of ARC3396 strain, the untreated sample of ARC3397 strain, the N-linked saccharide chain-treated sample of ARC3397 strain, and molecular marker (M).

As apparent from Fig. 12, the molecular weight of *β*-glucosidase 1 derived from *Aspergillus aculeatus* F-50 was decreased in ASP3396 strain and ASP3397 strain, both derived from saccharide chain-deficient strains, to 160,000 and 130,000, respectively. That is, by removing N-linked saccharide chain, each of the molecular weight of these three strains was decreased to 95,000. This result indicates that the differences in molecular weight were derived from the length of N-linked saccharide chain in each strain.

### (Thermostability)

With regard to the each purified sample, the thermostability test was carried out in accordance with the thermostability measurement method described in Test Example 7 at a temperature of from 50 to 75°C. The results are shown in Figure 13.

As apparent from Fig. 13, all the three stains, ASP3326 strain, ASP3396 strain, and ASP3397 strain, were found to be stable until 60°C, and there was no difference by the molecular weight.

### (Glucose inhibition)

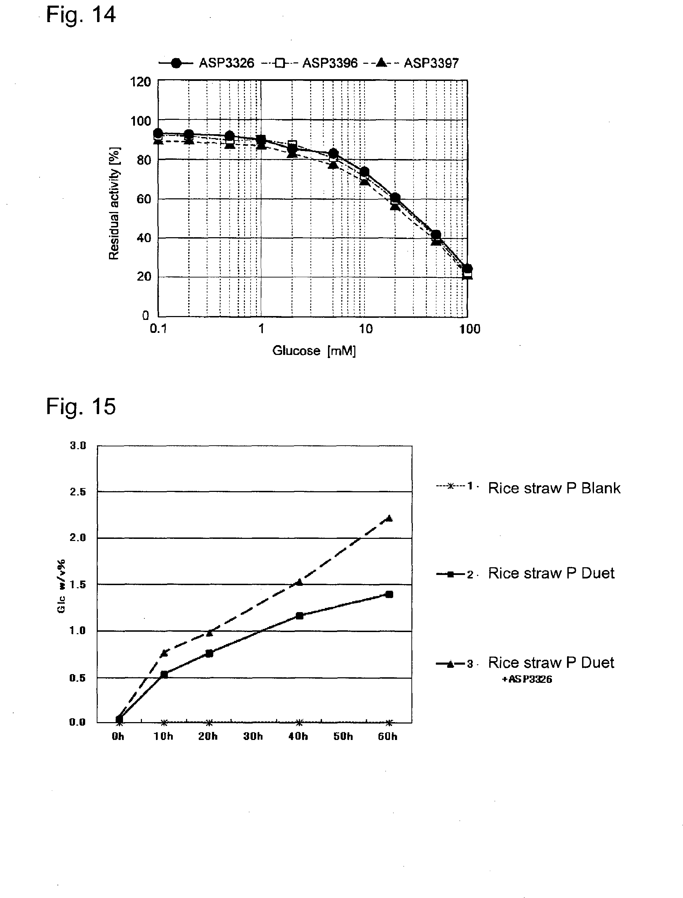

With regard to the each culture supernatant, the glucose inhibition activity was measured in accordance with the activity measurement method, by using 1 mM pNPG as a substrate and adding from 0 to 100 mM of glucose to the reaction mixture. The results are shown in Fig. 14.

As apparent from Fig. 14, all the three stains, ASP3326 strain, ASP3396 strain, and ASP3397 strain, showed the similar glucose inhibition activity, and therefore the glucose inhibition activity seemed to be not affected by the existence of a saccharide chain.

### [Test Example 12] Saccharification test of rice straw pulp and LBKP

By using a saccharification enzyme (BGL1) derived from each of ASP3336 strain, ASP3395 strain, and ASP3397 strain, which was purified in Test Example 11, and Accellerase DUET (cellulase manufactured by Genencor), the following saccharification test was carried out. Accellerase DUET was added in an amount of 5%(w/v) based on the dry weight of a pulp, and BGL1 was added in an amount of 10U per 1g of the dry weight of a pulp. The BGL1 was adjusted to 5U/ml before using based on pNPG activity

The pulp to be saccharified was produced as follows.

Australian Eucalyptus globules (8 years after planting) was employed as chips. Chips having a water content of 51 % were pulverized to a size of 2 cm x 2 cm, and then digested for 2 hours at 160°C with a liquor ratio of 4 and an active alkaline addition ratio of 17% to obtain an unbleached kraft pulp (UKP) with a kappa value of 20. The UKP was bleached in accordance with the following bleaching sequence. A four-stage bleaching comprised of an oxygen bleaching (O), a chlorine dioxide bleaching (D), an alkaline extraction + oxygen (E/O), and a chlorine dioxide bleaching (D) was employed as the bleaching sequence. The pulp concentration was kept to 10% during bleaching, and the addition ratio of each chemical was a ratio based on the pulp. Thus obtained bleached pulp was designated as LBKP, and subjected to a saccharification test. The bleaching conditions are shown in Table 7.

**[Table 7]**

| | | C | D | E/O | D |
|---|---|---|---|---|---|
| Pulp concentration (%) | | 10 | 10 | 10 | 10 |
| Bleaching temperature (°C) | | 100 | 70 | 70 | 70 |
| Bleaching time (min) | | 60 | 30 | 20 | 180 |
| Addition ratio of chemicals | Enzyme (MPa) | 0.5 | - | 0.15 | - |
| | NaOH (%) | 1.5 to 2.2 | | 1.1 | - |
| | ClO₂ (%) | - | 0.7 | - | 0.2 |

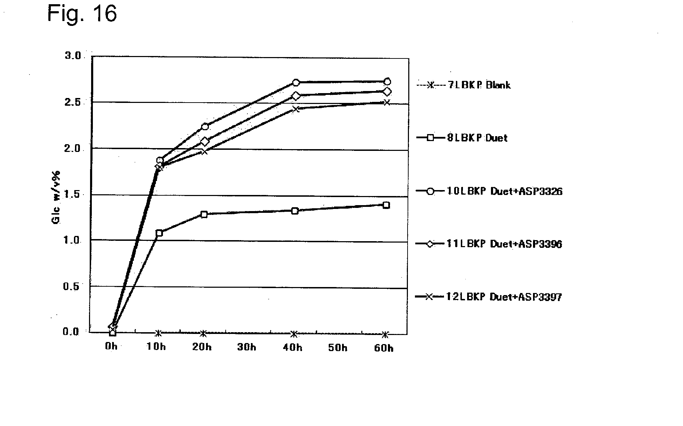

To a 100 ml plastic container having a lid, rice straw pulp or LBKP was introduced in an amount equivalent to its dry weight of 2.5g so that the pulp concentration became 5 wt%, and then 100 mM potassium phosphate buffer solution having a pH of 5.0 and a predetermined amount of an enzyme liquid were added thereto to achieve the total weight of 50.0g. 125 µl of Accellerase DUET and 5.0 ml of BGL1 or distilled water were added. The mixture was subjected to shaking at 150 rpm for 60 hours and maintained at 50°C, while collecting a mash along with the pulp at the time of 10 hours, 20 hours, 40 hours and 60 hours. The collected sample was centrifuged to obtain a supernatant as an assay sample. By means of an ion chromatography, the formed amount of monosaccharide was analyzed. As an ion chromatography apparatus, one having GP-40 gradient pump manufactured by Dionex corporation, AS-50 autosampler and PED detector was employed, and GP-40 pump was also used as a pump to feed 0.15M sodium hydroxide solution in a post-column method. The obtained data were analyzed by PeakNet Chromatography workstation manufactured by Dionex corporation. CarboPacPA 14 x 250 mm manufactured by Dionex corporation was used as the analysis column. As the standard sugar reagents, deoxyglucose and glucose manufactured by Kanto Chemical Co., Inc., were used. As the elution reagents, sodium hydroxide, potassium hydroxide and sodium carbonate manufactured by Kanto Chemical Co., Inc., were used. Further, Milli-Q water (Millipore) was employed as pure water. In the chromatography, pure water was used as eluent 1, 0.3N sodium hydroxide solution was used as eluent 2, 0.1 N potassium hydroxide solution was used as eluent 3, and 0.15N sodium carbonate solution was used as eluent 4. Further, in the post-column method, a Teflon tube having an inner diameter of 0.25 mm and a length of 5m was used to feed 0.15N sodium hydroxide solution at a flow rate of 0.5 ml/min, thereby to detect sugars stably. As the gradient for elution, pure water of eluent 1 was fed for 0.1 minute, then eluent 1 and eluent 2 were increased to 3% from 0.1 minute to 1.6 minute. From 1.6 minute to 2 minutes, eluent 1 was increased to 95% and eluent 2 was increased to 5%. Further, at 2.1 minutes, eluent 1 was increased to 100%, and then oligosaccharides and polysaccharides were eluted with 100% of eluent 1 until 9 minutes. Further, the column was cleaned-up with eluent 3 from 10.1 minutes to 10.6 minutes, and sufficiently washed with 30% of eluent 1 and 70% of eluent 4 for 0.1 minute, followed by equilibration in eluent 1 for 4.2 minutes to analyze the 15 minutes cycle. The results are shown in Table 8, Fig. 15 and Fig. 16.

**[Table 8]**

| | 0h | 10h | 20h | 40h | 60h |
|---|---|---|---|---|---|
| 1 Rice straw P Blank | ND | ND | ND | ND | ND |
| 2 Rice straw P Duet | 0.0 | 0.5 | 0.8 | 1.2 | 1.4 |
| 3 Rice straw P Duet+ASP3326 | 0.1 | 0.8 | 1.0 | 1.5 | 2.2 |
| 7 LBKP Blank | ND | ND | ND | ND | ND |
| 8 LBKP Duet | ND | 1.1 | 1.3 | 1.3 | 1.4 |
| 10 LBKP Duet+ASP3326 | 0.1 | 1.9 | 2.2 | 2.7 | 2.7 |
| 11 LBKP Duet+ASP3396 | 0.1 | 1.8 | 2.1 | 2.6 | 2.6 |
| 12 LBKP Duet+ASP3397 | 0.0 | 1.8 | 2.0 | 2.4 | 2.5 |

As shown in the first column, the second column and the third column of Table 8, and Fig. 15, with regard to rice straw pulp, BGL1 derived from ASP3326 strain was found to have an activity enhancement effect for Accellerase DUET. Further, as shown in the seventh column, the eighth column, the tenth column and the eleventh column of Table 8, and Fig. 16, with regard to LBKP, all of BGL1 derived from ASP3326 strain, ASP3396 strain and ASP3397 strain were found to have an activity enhancement effect for Accellerase DUET.

### INDUSTRIAL APPLICABILITY

By using the transformant of a yeast of the genus *Schizosaccharomyces* which produces a *β*-glucosidase derived from a filamentous fungus, it is possible to produce a *β-*glucosidase to be used for the enzymatic saccharification of cellulose, and further, by incubating the transformant which secretes the *β*-glucosidase in the presence of cellulose, it becomes possible to use cellulose for the enzymatic saccharification. Accordingly, the transformant of the present invention and the *β*-glucosidase produced therefrom may be used for producing biomass fuels including a sugar as a fermentation feedstock, a bioethanol, etc. from a cellulose type biomass.

The entire disclosure of Japanese Patent Application No. 2010-249092 filed on November 5, 2010, and Japanese Patent Application No. 2011-066540 filed on March 24, 2011 including specifications, claims and summaries are incorporated herein by reference in their entireties.

## Claims

1. A transformant of a yeast of the genus *Schizosaccharomyces* **characterized by** having a structural gene sequence encoding a *β*-glucosidase derived from a filamentous fungus, and a promoter sequence and a terminator sequence for expressing the structural gene in a chromosome, or alternatively by having the sequences as an extrachromosomal gene.

2. The transformant of a yeast of the genus *Schizosaccharomyces* according to Claim 1, wherein the *β*-glucosidase is BGL1.

3. The transformant of a yeast of the genus *Schizosaccharomyces* according to Claim 1 or 2, wherein the filamentous fungus is a microorganism of the genus *Aspergillus.*

4. The transformant of a yeast of the genus *Schizosaccharomyces* according to any one of Claims 1 to 3, wherein the *β*-glucosidase is comprised of an amino acid sequence of SEQ ID NO: 1, or is comprised of the amino acid sequence having deletion, substitution or addition of at least one amino acid, and has a catalytic activity to hydrolyze a *β*-D-glucopyranoside bond.

5. The transformant of a yeast of the genus *Schizosaccharomyces* according to any one of Claims 1 to 4, which further has a structural gene sequence of a secretion signal capable of functioning in the yeast of the genus *Schizosaccharomyces* at the 5' end side of the structural gene sequence encoding the *β*-glucosidase.

6. A transformation method for a yeast of the genus *Schizosaccharomyces,* **characterized in that** the yeast of the genus *Schizosaccharomyces* is transformed by using a vector having a structural gene sequence encoding a *β-*glucosidase derived from a filamentous fungus, and a promoter sequence and a terminator sequence for expressing the structural gene.

7. The transformation method for a yeast of the genus *Schizosaccharomyces* according to Claim 6, wherein a structural gene sequence of a secretion signal capable of functioning in the yeast of the genus *Schizosaccharomyces* is located at the 5' end side of the structural gene sequence encoding the *β*-glucosidase.

8. The transformation method for a yeast of the genus *Schizosaccharomyces* according to Claim 6 or 7, wherein the structural gene sequence encoding a *β-*glucosidase derived from a filamentous fungus is integrated into at least one position of a chromosome of the yeast of the genus *Schizosaccharomyces.*

9. The transformation method for a yeast of the genus *Schizosaccharomyces* according to Claim 8, wherein the gene sequence encoding the *β*-glucosidase is integrated into the chromosome by homologous recombination.

10. The transformation method for a yeast of the genus *Schizosaccharomyces* according to Claim 9, wherein the vector is integrated into a transposon gene Tf2 site.

11. A method for producing a *β*-glucosidase, **characterized in that** the *β-*glucosidase is recovered from cells obtained by incubating the transformant as defined in any one of Claims 1 to 4.

12. A method for producing a *β*-glucosidase, **characterized in that** the *β*-glucosidase is recovered from a culture broth obtained by incubating the transformant as defined in Claim 5.

13. A cellulose decomposition method, **characterized in that** the *β*-glucosidase obtained by the production method as defined in Claim 11 or 12 is used.

14. A cellulose decomposition method, **characterized in that** the transformant as defined in Claim 5 is cultivated in the presence of cellulose.
